# EUROPEAN PATENT APPLICATION

(11) **EP 4 781 997 A1**
(43) Date of publication of application: **29.07.2026**
(21) Application number: 25154554.7
(22) Date of filing: 28.01.2025
(51) Int. Cl.: A61K 35/741, A23L 33/135, A61K 31/047, A61K 35/747, C12N 1/20, A61K 35/00

(54) **LACTICASEIBACILLUS PARACASEI SUBSP. TOLERANS AND USES THEREOF**

(71) Applicant: Kerry Group Services International Limited, Tralee, County Kerry V92 EH11 (IE)
(72) Inventor: Bañuelos Hortigüela, Oscar, 18195 Cullar Vega (ES); Olivares Martín, Mónica, 18199 Cájar (ES); Blanco Rojo, Ruth, 28420 Galapagar (ES); Muñoz Ruiz, Manuel Jesus, 41013 Sevilla (ES); Gálvez Peralta, Julio Juan, 18012 Granada (ES); Molina Tijeras, Jose Alberto, 04867 Macael (ES); Rodriguez Nogales, Alba, 18198 Huetor Vega (ES); Rodríguez Cabezas, Maria Elena, 18005 Granada (ES)
(74) Representative: Bird & Bird Società tra Avvocati S.r.l.

(57) **Abstract**

The invention is in the field of biomedicine; and relates to *Lacticaseibacillus paracasei* subsp. *tolerans,* and a specific strain or a variant thereof, and their use in the treatment of insulin resistance syndrome, polycystic ovary syndrome, insulin resistance related infertility, gestational diabetes, obesity, and proinflammatory conditions. The invention also relates to a biologically pure culture, or a composition, or a pharmaceutical product, or a feed, or a nutritional product, or a nutraceutical composition comprising the specific strain or variant thereof, and a cosmetic method comprising administering the specific strain, or the biologically pure culture, composition, pharmaceutical product, feed, nutritional product, or nutraceutical composition.

## Description

### FIELD OF THE INVENTION

The present invention is comprised within the field of biomedicine. It specifically relates to a *Lacticaseibacillus paracasei* ssp. *tolerans* strain and it use in the treatment of pathologies associated with insulin resistant syndrome, infertility and proinflammatory conditions.

### BACKGROUND OF THE INVENTION

Insulin resistance is a state in which a given concentration of insulin produces a less-than-expected biological effect. Insulin resistance has also been arbitrarily defined as the requirement of 200 or more units of insulin per day to attain glycemic control and to prevent ketosis. The syndromes of insulin resistance actually make up a broad clinical spectrum, which includes obesity, glucose intolerance, diabetes, and the metabolic syndrome, as well as an extreme insulin-resistant state. Many of these disorders are associated with various endocrine, metabolic, and genetic conditions. These syndromes may also be associated with immunological diseases and may exhibit distinct phenotypic characteristics, such as obesity, cardiovascular disease, obesity-induced chronic inflammation, diabetes, metabolic syndrome, hypertension, central obesity, peripheral arterial disease, type A syndrome, type B syndrome, ancanthosis nigricans, polycystic ovary syndrome (PCOS), subfertility and other insulin-resistant states.

Polycystic ovary syndrome (PCOS) is an endocrine-metabolic disorder characterized by multiple hormonal imbalances, reflecting on a clinical presentation dominated by manifestations of hyperandrogenism, which generate short and long term consequences on female health. Among these, infertility is one of the most alarming associated morbidities, as it currently affects approximately 48.5 million women aged 20-44 years, with PCOS accounting for 6-15% of these cases, although up to 70% of women with PCOS may be undiagnosed. Indeed, its optimal diagnosis is often hindered due to its apparent similarities with several other pathologies remarkably, obesity as well as Cushing's syndrome, ovarian and adrenal neoplasms, and congenital adrenal hyperplasia.

The manifestations of PCOS are not confined to the gynecological sphere; women afflicted by this disease show an increased prevalence of several comorbidities, including obesity, dyslipidemia, hypertension, metabolic syndrome (MS), and type 2 diabetes mellitus (DM2) in comparison with women without PCOS. These features, along with other alterations such as endothelial dysfunction and a chronic low-grade inflammatory state, underlie the greater risk of developing cardiovascular disease and increased all-cause mortality observed in these subjects.

In this context, given the high prevalence of health problems related to insulin resistance syndrome nowadays, there is a great need for the identification of new strategies for the treatment and/or amelioration of the pathologies and/or symptoms associated with insulin resistance syndrome, especially those pathologies and/or symptoms related to women's reproductive health, inflammation, diabetes and obesity.

### SUMMARY OF THE INVENTION

The inventors have surprisingly found that the intake of *Lacticaseibacillus paracasei* subsp. *tolerans* is able to alleviate and improve some of the pathologies associated with the insulin resistance syndrome as well as other conditions such as infertility, PCOS, diabetes, gestational diabetes and other proinflammatory states. For example, the intake of the bacterial strain *Lacticaseibacillus paracasei* subsp. *tolerans* deposited in the CECT under Accession N° 9458 is able to alleviate and improve some of the pathologies associated with the insulin resistance syndrome as well as other conditions such as infertility, PCOS, diabetes, gestational diabetes and other proinflammatory states.

The bacterial strain *Lacticaseibacillus paracasei* subsp. *tolerans* was deposited as biological material under the terms of the Budapest Treaty on 24 October 2017; at the COLECCION ESPAÑOLA DE CULTIVOS TIPO (CECT), Edificio 3 CUE, Parc Cientific Universitat de Valencia, Catedrático Agustin Escardino, 9, 46980 Paterna (Valencia), ESPAÑA; and accorded accession number CECT 9458. This biological material accorded accession number CECT 9458 was deposited by Biosearch, S.A., Camino de Purchil 66, 18004 Granada, Spain. Accession number CECT 9458 refers to a bacterial strain which has genus *Lacticaseibacillus,* species *Lacticaseibacillus paracasei* and subspecies *tolerans.* Accession number CECT 9458 refers to a gram-positive, homofermentative, non-spore forming, rod shaped bacterial strain. Accession number CECT 9458 refers to a bacterial strain that has optimal growth conditions on solid medium of incubation at 37 °C for 48 h on De Man, Rogosa and Sharpe (MRS) agar medium and in an anoxic atmosphere. The bacterial strain deposited under accession number CECT 9458 can also be grown in liquid medium using MRS broth and at 37°C without shaking. Further information about the bacterial strain deposited under accession number CECT 9458 is described throughout the present application, such as in Example 1.

The terms "CECT 9458" and "9458" are used herein synonymously to refer to the same accession number.

Thus, in a first aspect, the invention relates to a bacterial strain of *Lacticaseibacillus paracasei* subsp. *tolerans* deposited in the CECT under Accession N° 9458, or a variant thereof.

Optionally, the invention relates to a composition comprising the bacterial strain of *Lacticaseibacillus paracasei* subsp. *tolerans* deposited in the CECT under Accession N° 9458, or a variant thereof. Optionally, the composition further comprises D-chiro-inositol.

Optionally, the bacterial strain is alive.

Alternatively, the bacterial strain is inactivated, optionally partially inactivated, optionally totally inactivated. Optionally, the bacterial strain has been inactivated by an inactivation process selected from the group consisting of: thermal inactivation, microwave inactivation, pressure inactivation, acid inactivation, base inactivation, ethanol inactivation and peroxide inactivation.

Optionally, the bacterial strain is a probiotic strain.

Optionally, the bacterial strain comprises a 16S rRNA gene sequence comprising a nucleotide sequence that is at least 70% identical to the nucleotide sequence of SEQ ID NO: 1. Optionally, the bacterial strain comprises a 16S rRNA gene sequence comprising a nucleotide sequence that is at least 80% identical to the nucleotide sequence of SEQ ID NO: 1. Optionally, the bacterial strain comprises a 16S rRNA gene sequence comprising a nucleotide sequence that is at least 90% identical to the nucleotide sequence of SEQ ID NO: 1. Optionally, the bacterial strain comprises a 16S rRNA gene sequence comprising a nucleotide sequence that is at least 95% identical to the nucleotide sequence of SEQ ID NO: 1. Optionally, the bacterial strain comprises a 16S rRNA gene sequence comprising a nucleotide sequence that is 100% identical to the nucleotide sequence of SEQ ID NO: 1. Optionally, the bacterial strain comprises a 16S rRNA gene sequence comprising a nucleotide sequence that is identical to the nucleotide sequence of SEQ ID NO: 1.

Optionally, the bacterial strain comprises a 16S rRNA gene sequence consisting of a nucleotide sequence that is at least 70% identical to the nucleotide sequence of SEQ ID NO: 1. Optionally, the bacterial strain comprises a 16S rRNA gene sequence consisting of a nucleotide sequence that is at least 80% identical to the nucleotide sequence of SEQ ID NO: 1. Optionally, the bacterial strain comprises a 16S rRNA gene sequence consisting of a nucleotide sequence that is at least 90% identical to the nucleotide sequence of SEQ ID NO: 1. Optionally, the bacterial strain comprises a 16S rRNA gene sequence consisting of a nucleotide sequence that is at least 95% identical to the nucleotide sequence of SEQ ID NO: 1. Optionally, the bacterial strain comprises a 16S rRNA gene sequence consisting of a nucleotide sequence that is 100% identical to the nucleotide sequence of SEQ ID NO: 1. Optionally, the bacterial strain comprises a 16S rRNA gene sequence consisting of a nucleotide sequence that is identical to the nucleotide sequence of SEQ ID NO: 1.

Optionally, the invention relates to a food supplement comprising a bacterial strain of *Lacticaseibacillus paracasei* subsp. *tolerans* deposited in the CECT under Accession N° 9458, or a variant thereof. Optionally, the food supplement further comprises D-chiro-inositol. Optionally, the food supplement is for non-therapeutic use.

In a second aspect, the invention relates to a bacterial strain of *Lacticaseibacillus paracasei* subsp. *tolerans* comprising a 16S rRNA gene sequence comprising a nucleotide sequence that is at least 70% identical to the nucleotide sequence of SEQ ID NO: 1.

Optionally, the bacterial strain comprises a 16S rRNA gene sequence comprising a nucleotide sequence that is at least 80% identical to the nucleotide sequence of SEQ ID NO: 1. Optionally, the bacterial strain comprises a 16S rRNA gene sequence comprising a nucleotide sequence that is at least 90% identical to the nucleotide sequence of SEQ ID NO: 1. Optionally, the bacterial strain comprises a 16S rRNA gene sequence comprising a nucleotide sequence that is at least 95% identical to the nucleotide sequence of SEQ ID NO: 1. Optionally, the bacterial strain comprises a 16S rRNA gene sequence comprising a nucleotide sequence that is 100% identical to the nucleotide sequence of SEQ ID NO: 1. Optionally, the bacterial strain comprises a 16S rRNA gene sequence comprising a nucleotide sequence that is identical to the nucleotide sequence of SEQ ID NO: 1.

Optionally, the bacterial strain comprises a 16S rRNA gene sequence consisting of a nucleotide sequence that is at least 70% identical to the nucleotide sequence of SEQ ID NO: 1. Optionally, the bacterial strain comprises a 16S rRNA gene sequence consisting of a nucleotide sequence that is at least 80% identical to the nucleotide sequence of SEQ ID NO: 1. Optionally, the bacterial strain comprises a 16S rRNA gene sequence consisting of a nucleotide sequence that is at least 90% identical to the nucleotide sequence of SEQ ID NO: 1. Optionally, the bacterial strain comprises a 16S rRNA gene sequence consisting of a nucleotide sequence that is at least 95% identical to the nucleotide sequence of SEQ ID NO: 1. Optionally, the bacterial strain comprises a 16S rRNA gene sequence consisting of a nucleotide sequence that is 100% identical to the nucleotide sequence of SEQ ID NO: 1. Optionally, the bacterial strain comprises a 16S rRNA gene sequence consisting of a nucleotide sequence that is identical to the nucleotide sequence of SEQ ID NO: 1.

As used herein, unless stated otherwise, "bacterial strain of the invention" or simply "the bacterial strain" refers to any bacterial strain disclosed in the present application, including the bacterial strains of the first and second aspect of the present invention described above.

In a third aspect, the invention relates to a biologically pure culture, or a composition, or a pharmaceutical product, or a feed, or a nutritional product, or a nutraceutical composition, comprising the bacterial strain of the invention.

Optionally, the biologically pure culture, composition, pharmaceutical product, feed, nutritional product, or nutraceutical composition, further comprises D-chiro-inositol.

Optionally, the bacterial strain, or the biologically pure culture, composition, pharmaceutical product, feed, nutritional product, or nutraceutical composition is frozen, lyophilized or dried. Optionally, the bacterial strain, or the biologically pure culture, composition, pharmaceutical product, feed, nutritional product, or a nutraceutical composition, is in a frozen, lyophilized or dried form.

In a fourth aspect, the invention relates to a bacterial strain of *Lacticaseibacillus paracasei* subsp. *tolerans* for use as a medicament.

Optionally, the invention relates to a composition comprising a bacterial strain of *Lacticaseibacillus paracasei* subsp. *tolerans* for use as a medicament. Optionally, the composition for use as a medicament further comprises D-chiro-inositol.

Optionally, the bacterial strain for use as a medicament is alive. Alternatively, the bacterial strain for use as a medicament is inactivated, optionally partially inactivated, optionally totally inactivated. Optionally, the bacterial strain for use as a medicament has been inactivated by an inactivation process selected from the group consisting of: thermal inactivation, microwave inactivation, pressure inactivation, acid inactivation, base inactivation, ethanol inactivation and peroxide inactivation.

Optionally, the bacterial strain for use as a medicament is a probiotic strain.

Optionally, the bacterial strain for use as a medicament is combined with D-chiro-inositol. Optionally, the bacterial strain is administered to a subject together with D-chiro-inositol. Optionally, the bacterial strain is administered to a subject in combination with D-chiro-inositol. Optionally, the invention relates to a composition for use as a medicament, wherein the composition comprises a bacterial strain of *Lacticaseibacillus paracasei* subsp. *tolerans* and D-chiro-inositol. Optionally, the invention relates to a composition for use as a medicament, wherein the composition comprises D-chiro-inositol and the bacterial strain of *Lacticaseibacillus paracasei* subsp. *tolerans* deposited in the CECT under Accession N° 9458, or a variant thereof.

Optionally, the bacterial strain for use as a medicament is the bacterial strain of the first or second aspect of the present invention.

Optionally, the bacterial strain for use as a medicament is the bacterial strain *Lacticaseibacillus paracasei* subsp. *tolerans* deposited in the CECT under Accession N° 9458, or a variant thereof.

Optionally, the bacterial strain for use as a medicament is the bacterial strain *Lacticaseibacillus paracasei* subsp. *tolerans* deposited in the CECT under Accession N° 9458, or a variant thereof; wherein the bacterial strain is alive. Alternatively, the bacterial strain for use as a medicament is the bacterial strain *Lacticaseibacillus paracasei* subsp. *tolerans* deposited in the CECT under Accession N° 9458, or a variant thereof; wherein the bacterial strain is inactivated, optionally partially inactivated, optionally totally inactivated. Optionally, the bacterial strain for use as a medicament is the bacterial strain *Lacticaseibacillus paracasei* subsp. *tolerans* deposited in the CECT under Accession N° 9458, or a variant thereof; wherein the bacterial strain has been inactivated by an inactivation process selected from the group consisting of: thermal inactivation, microwave inactivation, pressure inactivation, acid inactivation, base inactivation, ethanol inactivation and peroxide inactivation.

Optionally, the bacterial strain for use as a medicament is the bacterial strain *Lacticaseibacillus paracasei* subsp. *tolerans* deposited in the CECT under Accession N° 9458, or a variant thereof; wherein the bacterial strain is a probiotic strain.

Optionally, the bacterial strain for use as a medicament comprises a 16S rRNA gene sequence comprising a nucleotide sequence that is at least 70%, 80%, 90%, 95% or 100% identical to the nucleotide sequence of SEQ ID NO: 1. Optionally, the bacterial strain for use as a medicament comprises a 16S rRNA gene sequence comprising a nucleotide sequence that is identical to the nucleotide sequence of SEQ ID NO: 1.

Optionally, the bacterial strain for use as a medicament comprises a 16S rRNA gene sequence consisting of a nucleotide sequence that is at least 70%, 80%, 90%, 95%, or 100% identical to the nucleotide sequence of SEQ ID NO: 1. Optionally, the bacterial strain for use as a medicament comprises a 16S rRNA gene sequence consisting of a nucleotide sequence that is identical to the nucleotide sequence of SEQ ID NO: 1.

Optionally, the bacterial strain for use as a medicament is for use in the treatment and/or prevention of insulin resistance related infertility in a female subject; optionally wherein the insulin resistance related infertility is associated with diabetes, polycystic ovary syndrome and/or with insulin resistance syndrome. Optionally, the bacterial strain for use as a medicament is for use in the treatment and/or prevention of a condition selected from the group consisting of: gestational diabetes, insulin resistance syndrome, type 2 diabetes, obesity, heart disease, high blood pressure, proinflammatory state, proinflammatory condition, and polycystic ovary syndrome.

Optionally, the bacterial strain for use as a medicament is a bacterial strain of *Lacticaseibacillus paracasei* subsp. *tolerans;* wherein the bacterial strain is for use in the treatment and/or prevention of insulin resistance related infertility in a female subject; optionally wherein the insulin resistance related infertility is associated with diabetes, polycystic ovary syndrome and/or with insulin resistance syndrome. Optionally, the bacterial strain for use as a medicament is a bacterial strain of *Lacticaseibacillus paracasei* subsp. *tolerans;* wherein the bacterial strain is for use in the treatment and/or prevention of a condition selected from the group consisting of: gestational diabetes, insulin resistance syndrome, type 2 diabetes, obesity, heart disease, high blood pressure, proinflammatory state, proinflammatory condition, and polycystic ovary syndrome.

Optionally, the bacterial strain for use as a medicament is for use in the treatment and/or prevention of polycystic ovary syndrome; wherein the bacterial strain is a bacterial strain of *Lacticaseibacillus paracasei* subsp. *tolerans.* Optionally, the bacterial strain for use in the treatment and/or prevention of polycystic ovary syndrome is combined with D-chiro-inositol. Optionally, the invention relates to a composition for use in the treatment and/or prevention of polycystic ovary syndrome, wherein the composition comprises D-chiro-inositol and a bacterial strain of *Lacticaseibacillus paracasei* subsp. *tolerans.*

Optionally, the bacterial strain for use as a medicament is the bacterial strain of *Lacticaseibacillus paracasei* subsp. *tolerans* deposited in the CECT under Accession N° 9458, or a variant thereof; wherein the bacterial strain is for use in the treatment and/or prevention of insulin resistance related infertility in a female subject; optionally wherein the insulin resistance related infertility is associated with diabetes, polycystic ovary syndrome and/or with insulin resistance syndrome. Optionally, the bacterial strain for use as a medicament is the bacterial strain of *Lacticaseibacillus paracasei* subsp. *tolerans* deposited in the CECT under Accession N° 9458, or a variant thereof; wherein the bacterial strain is for use in the treatment and/or prevention of a condition selected from the group consisting of: gestational diabetes, insulin resistance syndrome, type 2 diabetes, obesity, heart disease, high blood pressure, proinflammatory state, proinflammatory condition, and polycystic ovary syndrome.

Optionally, the bacterial strain for use as a medicament is for use in the treatment and/or prevention of polycystic ovary syndrome; wherein the bacterial strain is the bacterial strain of *Lacticaseibacillus paracasei* subsp. *tolerans* deposited in the CECT under Accession N° 9458, or a variant thereof. Optionally, the bacterial strain for use in the treatment and/or prevention of polycystic ovary syndrome is combined with D-chiro-inositol. Optionally, the invention relates to a composition for use in the treatment and/or prevention of polycystic ovary syndrome, wherein the composition comprises D-chiro-inositol and the bacterial strain of *Lacticaseibacillus paracasei* subsp. *tolerans* deposited in the CECT under Accession N° 9458, or a variant thereof.

The invention also relates to the biologically pure culture, composition, pharmaceutical product, feed, nutritional product, or nutraceutical composition of the invention for use as a medicament.

The invention also relates to the bacterial strain, or the biologically pure culture, composition, pharmaceutical product, feed, nutritional product, or nutraceutical composition of the invention; for use in the treatment and/or prevention of polycystic ovary syndrome. Optionally, the bacterial strain is a probiotic strain.

The invention also relates to the bacterial strain, or the biologically pure culture, composition, pharmaceutical product, feed, nutritional product, or nutraceutical composition of the invention; for use in the treatment and/or prevention of insulin resistance related infertility in a female subject. Optionally, the bacterial strain is a probiotic strain.

The invention also relates to the bacterial strain, or the biologically pure culture, composition, pharmaceutical product, feed, nutritional product, or nutraceutical composition of the invention; for use in the treatment and/or prevention of gestational diabetes. Optionally, the bacterial strain is a probiotic strain.

The invention also relates to the bacterial strain, or the biologically pure culture, composition, pharmaceutical product, feed, nutritional product, or a nutraceutical composition of the invention; for use in the treatment and/or prevention of resistance insulin syndrome and related conditions selected from type 2 diabetes, obesity, heart disease and high blood pressure. Optionally, the bacterial strain is a probiotic strain.

The invention also relates to the bacterial strain, or the biologically pure culture, composition, pharmaceutical product, feed, nutritional product, or nutraceutical composition of the invention; for use in the treatment and/or prevention of proinflammatory states or conditions. Optionally, the bacterial strain is a probiotic strain.

The invention also relates to a method of treatment and/or prevention of a disease or condition; wherein the method comprises the administration to a subject of the bacterial strain, or the biologically pure culture, composition, feed, nutritional product, or nutraceutical composition of the invention. Optionally, the disease or condition is insulin resistance related infertility in a female subject; optionally the insulin resistance related infertility is associated with diabetes, polycystic ovary syndrome and/or with insulin resistance syndrome. Optionally, the disease or condition is selected from the group consisting of: gestational diabetes, insulin resistance syndrome, type 2 diabetes, obesity, heart disease, high blood pressure, proinflammatory state, proinflammatory condition, and polycystic ovary syndrome.

Optionally, the method of treatment and/or prevention comprises the administration to a subject of a bacterial strain of *Lacticaseibacillus paracasei* subsp. *tolerans;* optionally in combination with D-chiro-inositol.

Optionally, the method of treatment and/or prevention comprises the administration to a subject of the bacterial strain of *Lacticaseibacillus paracasei* subsp. *tolerans* deposited in the CECT under Accession N° 9458, or a variant thereof; optionally in combination with D-chiro-inositol.

Optionally, the method of treatment and/or prevention comprises the administration to a subject of a bacterial strain comprising a 16S rRNA gene sequence comprising a nucleotide sequence that is at least 70%, 80%, 90%, 95% or 100% identical to the nucleotide sequence of SEQ ID NO: 1. Optionally, the method of treatment and/or prevention comprises the administration to a subject of a bacterial strain comprising a 16S rRNA gene sequence comprising a nucleotide sequence that is identical to the nucleotide sequence of SEQ ID NO: 1. Optionally, the method of treatment and/or prevention comprises the administration to a subject of a bacterial strain comprising a 16S rRNA gene sequence consisting of a nucleotide sequence that is at least 70%, 80%, 90%, 95% or 100% identical to the nucleotide sequence of SEQ ID NO: 1. Optionally, the method of treatment and/or prevention comprises the administration to a subject of a bacterial strain comprising a 16S rRNA gene sequence consisting of a nucleotide sequence that is identical to the nucleotide sequence of SEQ ID NO: 1.

Optionally, the bacterial strain is a probiotic strain. Optionally, the bacterial strain is alive. Alternatively, the bacterial strain is inactivated, optionally partially inactivated, optionally totally inactivated. Optionally, the bacterial strain has been inactivated by an inactivation process selected from the group consisting of: thermal inactivation, microwave inactivation, pressure inactivation, acid inactivation, base inactivation, ethanol inactivation and peroxide inactivation.

The invention also relates to use of a composition in the manufacture of a medicament for treatment and/or prevention of a disease or condition; wherein the composition comprises the bacterial strain, or the biologically pure culture, composition, feed, nutritional product, or nutraceutical composition of the invention. Optionally, the disease or condition is insulin resistance related infertility in a female subject; optionally the insulin resistance related infertility is associated with diabetes, polycystic ovary syndrome and/or with insulin resistance syndrome. Optionally, the disease or condition is selected from the group consisting of: gestational diabetes, insulin resistance syndrome, type 2 diabetes, obesity, heart disease, high blood pressure, proinflammatory state, proinflammatory condition, and polycystic ovary syndrome.

Optionally, the composition comprises a bacterial strain of *Lacticaseibacillus paracasei* subsp. *tolerans;* optionally in combination with D-chiro-inositol.

Optionally, the composition comprises the bacterial strain of *Lacticaseibacillus paracasei* subsp. *tolerans* deposited in the CECT under Accession N° 9458, or a variant thereof; optionally in combination with D-chiro-inositol.

Optionally, the composition comprises a bacterial strain comprising a 16S rRNA gene sequence comprising a nucleotide sequence that is at least 70%, 80%, 90%, 95% or 100% identical to the nucleotide sequence of SEQ ID NO: 1. Optionally, the composition comprises a bacterial strain comprising a 16S rRNA gene sequence comprising a nucleotide sequence that is identical to the nucleotide sequence of SEQ ID NO: 1.

Optionally, the composition comprises a bacterial strain comprising a 16S rRNA gene sequence consisting of a nucleotide sequence that is at least 70%, 80%, 90%, 95%, or 100% identical to the nucleotide sequence of SEQ ID NO: 1. Optionally, the composition comprises a bacterial strain comprising a 16S rRNA gene sequence consisting of a nucleotide sequence that is identical to the nucleotide sequence of SEQ ID NO: 1.

Optionally, the bacterial strain is a probiotic strain. Optionally, the bacterial strain is alive. Alternatively, the bacterial strain is inactivated, optionally partially inactivated, optionally totally inactivated. Optionally, the bacterial strain has been inactivated by an inactivation process selected from the group consisting of: thermal inactivation, microwave inactivation, pressure inactivation, acid inactivation, base inactivation, ethanol inactivation and peroxide inactivation.

In a further aspect, the invention relates to a cosmetic method for the treatment of obesity comprising the administration to a subject of the bacterial strain of the invention, or the biologically pure culture, composition, feed, nutritional product, or nutraceutical composition of the invention. Optionally, the bacterial strain is a probiotic strain.

Optionally, the cosmetic method for the treatment of obesity comprises the administration to a subject of a bacterial strain; wherein the bacterial strain is alive. Alternatively, the cosmetic method for the treatment of obesity comprises the administration to a subject of a bacterial strain; wherein the bacterial strain is inactivated, optionally partially inactivated, optionally totally inactivated. Optionally, the cosmetic method for the treatment of obesity comprises the administration to a subject of a bacterial strain; wherein the bacterial strain has been inactivated by an inactivation process selected from the group consisting of: thermal inactivation, microwave inactivation, pressure inactivation, acid inactivation, base inactivation, ethanol inactivation and peroxide inactivation.

Optionally, the cosmetic method for the treatment of obesity comprises the administration to a subject of a bacterial strain of *Lacticaseibacillus paracasei* subsp. *tolerans.*

Optionally, the cosmetic method for the treatment of obesity comprises the administration to a subject of a bacterial strain of *Lacticaseibacillus paracasei* subsp. *tolerans* comprising a 16S rRNA gene sequence comprising a nucleotide sequence that is at least 70%, 80%, 90%, 95% or 100% identical to the nucleotide sequence of SEQ ID NO: 1. Optionally, the cosmetic method for the treatment of obesity comprises the administration to a subject of a bacterial strain of *Lacticaseibacillus paracasei* subsp. *tolerans* comprising a 16S rRNA gene sequence comprising a nucleotide sequence that is identical to the nucleotide sequence of SEQ ID NO: 1.

Optionally, the cosmetic method for the treatment of obesity comprises the administration to a subject of a bacterial strain of *Lacticaseibacillus paracasei* subsp. *tolerans* comprising a 16S rRNA gene sequence consisting of a nucleotide sequence that is at least 70%, 80%, 90%, 95%, or 100% identical to the nucleotide sequence of SEQ ID NO: 1. Optionally, the cosmetic method for the treatment of obesity comprises the administration to a subject of a bacterial strain of *Lacticaseibacillus paracasei* subsp. *tolerans* comprising a 16S rRNA gene sequence consisting of a nucleotide sequence that is identical to the nucleotide sequence of SEQ ID NO: 1.

Optionally, the cosmetic method for the treatment of obesity comprises the administration to a subject of the bacterial strain of *Lacticaseibacillus paracasei* subsp. *tolerans* deposited in the CECT under Accession N° 9458, or a variant thereof.

Optionally, the bacterial strain is combined with D-chiro-inositol. Optionally, the cosmetic method for the treatment of obesity comprises the administration to a subject of a bacterial strain of *Lacticaseibacillus paracasei* subsp. *tolerans* combined with D-chiro-inositol. Optionally, the cosmetic method for the treatment of obesity comprises the administration to a subject of a bacterial strain of *Lacticaseibacillus paracasei* subsp. *tolerans* together with D-chiro-inositol. Optionally, the cosmetic method for the treatment of obesity comprises the administration to a subject of a bacterial strain of *Lacticaseibacillus paracasei* subsp. *tolerans* in combination with D-chiro-inositol. Optionally, the cosmetic method for the treatment of obesity comprises the administration to a subject of a composition comprising D-chiro-inositol and a bacterial strain of *Lacticaseibacillus paracasei* subsp. *tolerans.*

Optionally, the bacterial strain is combined with D-chiro-inositol. Optionally, the cosmetic method for the treatment of obesity comprises the administration to a subject of the bacterial strain of *Lacticaseibacillus paracasei* subsp. *tolerans* deposited in the CECT under Accession N° 9458, or a variant thereof, combined with D-chiro-inositol. Optionally, the cosmetic method for the treatment of obesity comprises the administration to a subject of the bacterial strain of *Lacticaseibacillus paracasei* subsp. *tolerans* deposited in the CECT under Accession N° 9458, or a variant thereof, together with D-chiro-inositol. Optionally, the cosmetic method for the treatment of obesity comprises the administration to a subject of the bacterial strain of *Lacticaseibacillus paracasei* subsp. *tolerans* deposited in the CECT under Accession N° 9458, or a variant thereof, in combination with D-chiro-inositol. Optionally, the cosmetic method for the treatment of obesity comprises the administration to a subject of a composition comprising D-chiro-inositol and the bacterial strain of *Lacticaseibacillus paracasei* subsp. *tolerans* deposited in the CECT under Accession N° 9458, or a variant thereof.

### DESCRIPTION OF THE FIGURES

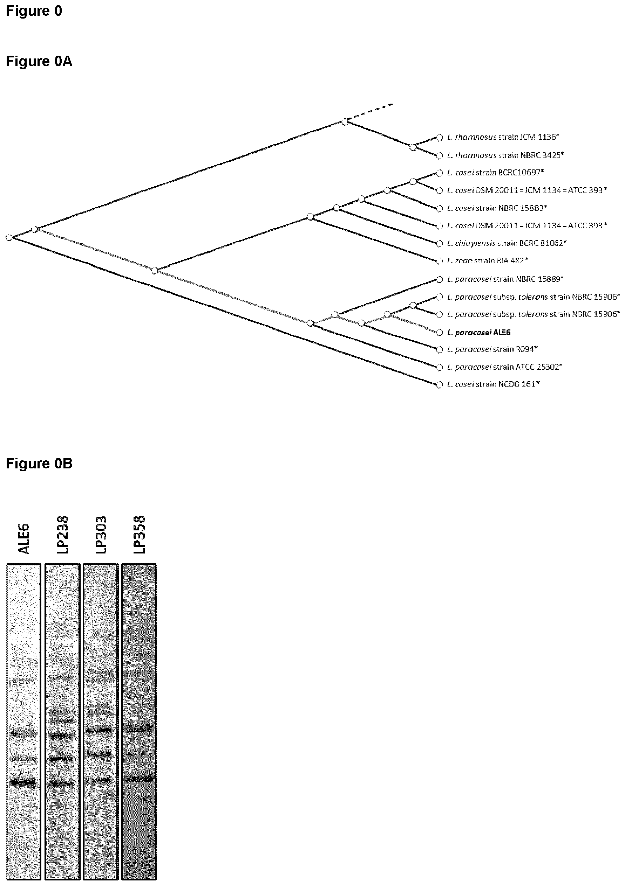

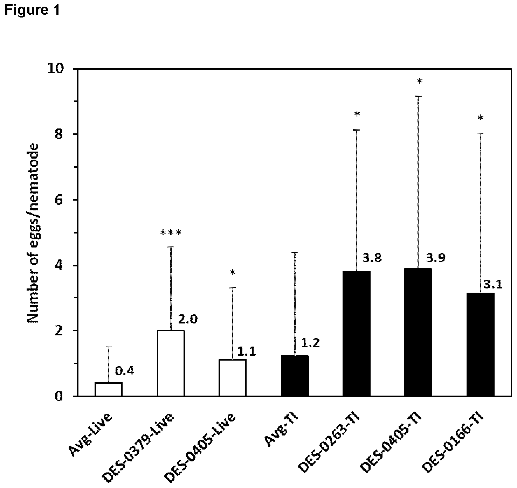

**Figure 0****.** Characterization of the strain of *Lacticaseibacillus paracasei* deposited under accession number CECT 9458, also called *L. paracasei* ALE6. A) Distance tree of BLAST pairwise alignments of 16S rRNA gene sequence of *L. paracasei* ALE6 and closely related type microorganisms. *Partial sequence. B) DNA fingerprint obtained by ribotyping of *L. paracasei* ALE6 and 3 *L. paracasei* from the Kerry microorganism collection: LP238, LP303 and LP358.**Figure 1****.** Average egg-laying for the age-1 strain - (N=10) grown from different alive (white bars) and thermally inactivated bacteria (black bars). t-Student test (*p-value < 0.05; *** p-value < 0,0001). The reference values set as control are obtained from the average for alive and thermally inactivated bacteria independently. The bacteria have been washed with saline solution to remove the glycerol.

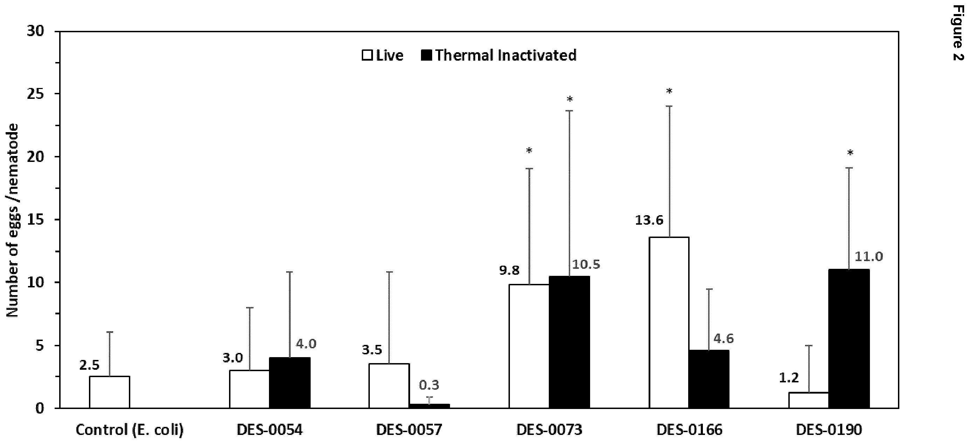

**Figure 2****.** Average egg-laying of the age-1 strain (mg305) (N=10) grown from different alive and thermally inactivated bacteria. Bacteria have been washed with saline solution to remove the glycerol. t-Student test (*p-value < 0.05).

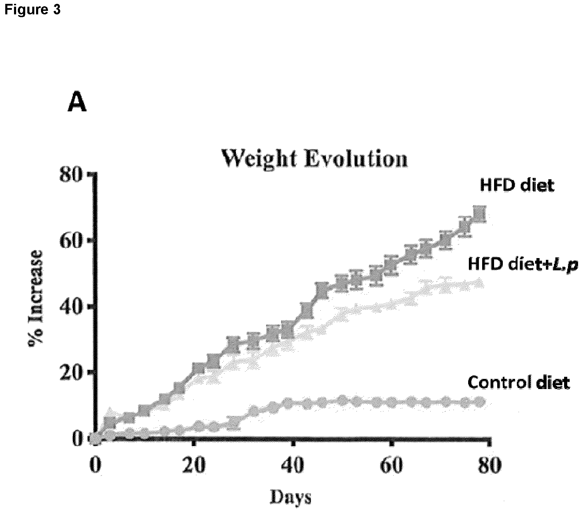

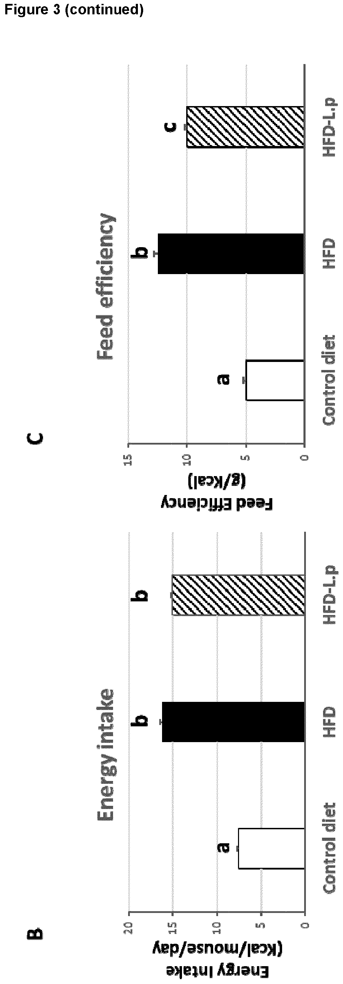

**Figure 3****.** Effects of *L*. *paracasei* subsp. *tolerans* (CECT accession N° 9458) administration on body weight evolution and energy intake. A) Administration of *L. paracasei* reduced weight gain during all the experiment compared with non-treated mice. B) Energy intake of high-fat diet (HFD)-fed mice were similar, however C) the feed efficiency of treated mice was significantly reduced. Data are expressed as means ± SEM (n = 10). *P < 0.05 vs. HFD-fed mice. Groups with different letter statistically differ (P < 0.05).

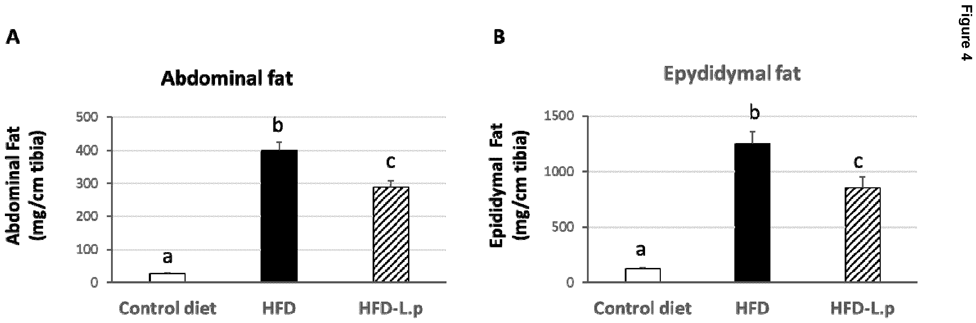

**Figure 4****.** Effects of *L. paracasei* subsp. *tolerans* (CECT accession N° 9458) on fats deposits. A) Administration of the bacterial strain reduced abdominal and B) epididymal fat deposits compared with non-treated mice. Data are expressed as means ±SEM (n = 10). Groups with different letter statistically differ (P < 0.05).

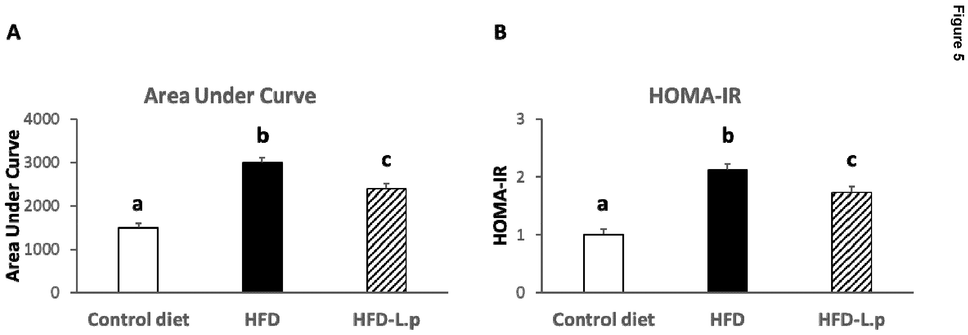

**Figure 5**. Effects of *L*. *paracasei* subsp. *tolerans* (CECT accession N° 9458) on glucose tolerance. A) Treated mice exhibited a lower area under the curve (AUC) obtained from the glucose tolerance test. B) It was reflected in a lower insulin resistance indicated by HOMA-IR parameter. Data are expressed as means ± SEM (n = 10). Groups with different letter statistically differ (P < 0.05).

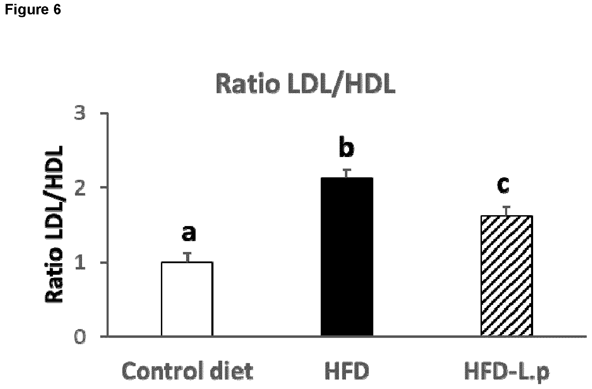

**Figure 6**. Effects of *L*. *paracasei* subsp. *tolerans* (CECT accession N° 9458) on cholesterol metabolism. Data are expressed as means ±SEM (n = 10). Groups with different letter statistically differ (P < 0.05).

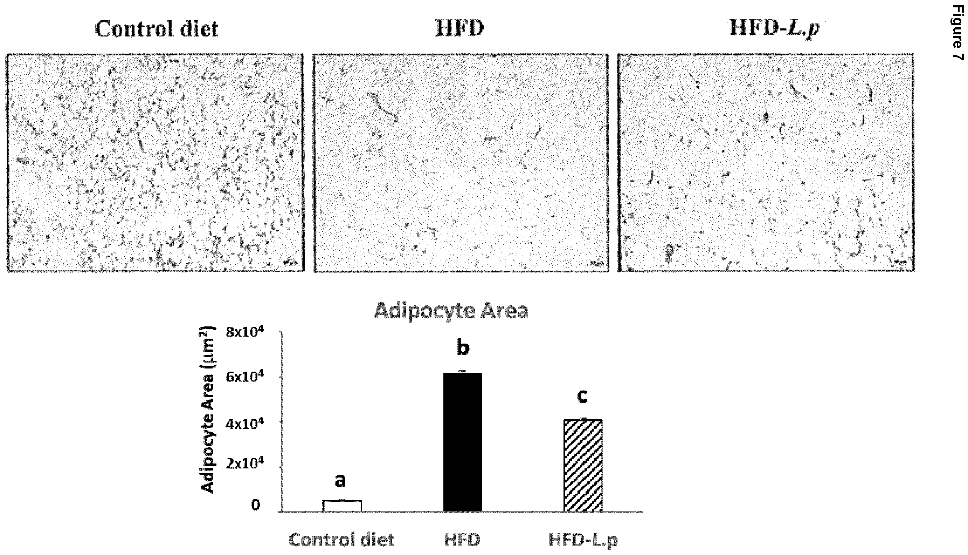

**Figure 7**. Epididymal adipose tissue, analysed by hematoxylin and eosin staining (scale bar = 50 µm) and on adipocyte area. Data are expressed as means ± SEM (n = 10). Groups with different letter statistically differ (P < 0.05).

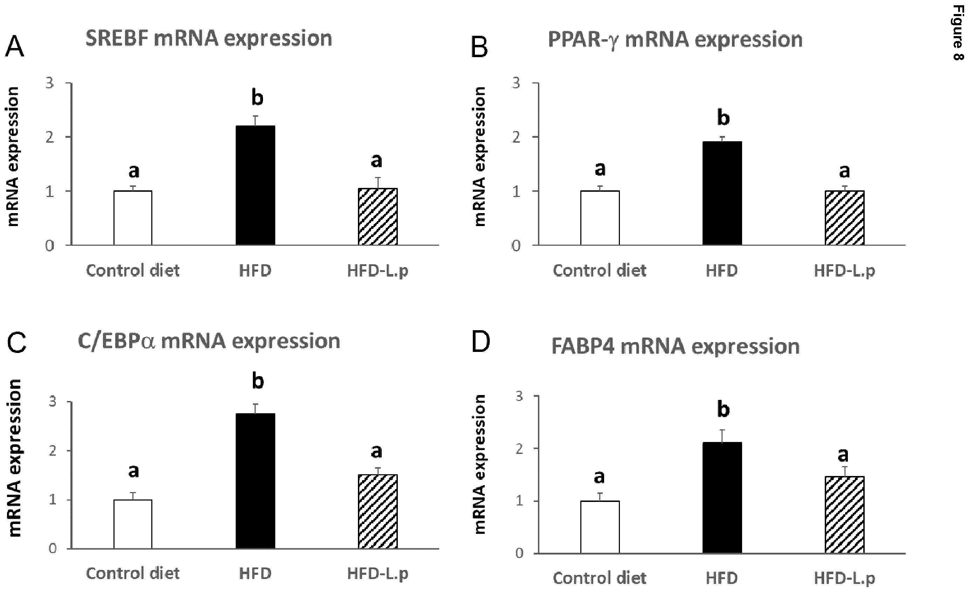

**Figure 8**. Effects of *L*. *paracasei*subsp. *tolerans* (CECT accession N° 9458) on adipogenesis gene expression in epididymal adipose tissue. A) *Srebf1,* B) *Ppar-y,* C) *Cebpa* and D) *Fabp4.*
Data are expressed as means ± SEM (n = 10). Groups with different letter statistically differ (P < 0.05).

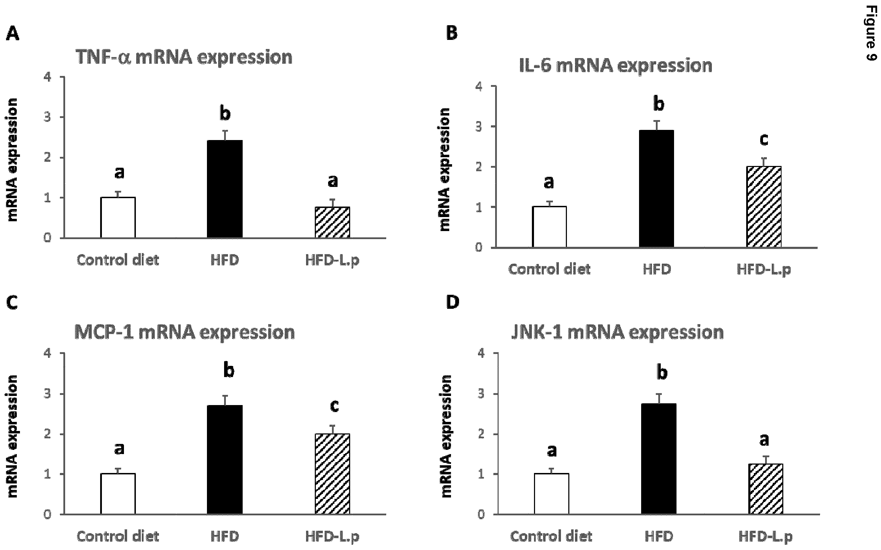

**Figure 9**. Effects of *L*. paracaseisubsp. *tolerans* (CECT accession N° 9458) on pro-inflammatory marker gene expression in epididymal adipose tissue. HFD promoted increased expression levels of A) *Tnfα,* B) II6, C) *Mcp1* and D) *Jnk1.* The expression of all these markers were reduced with the administration of *L. paracasei.* Data are expressed as means ±SEM (n = 10). Groups with different letter statistically differ (P < 0.05).

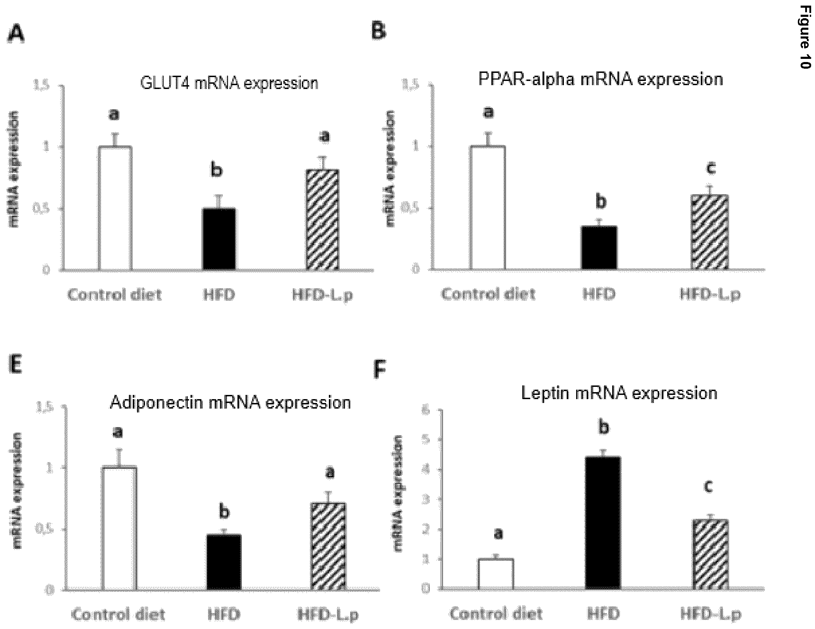

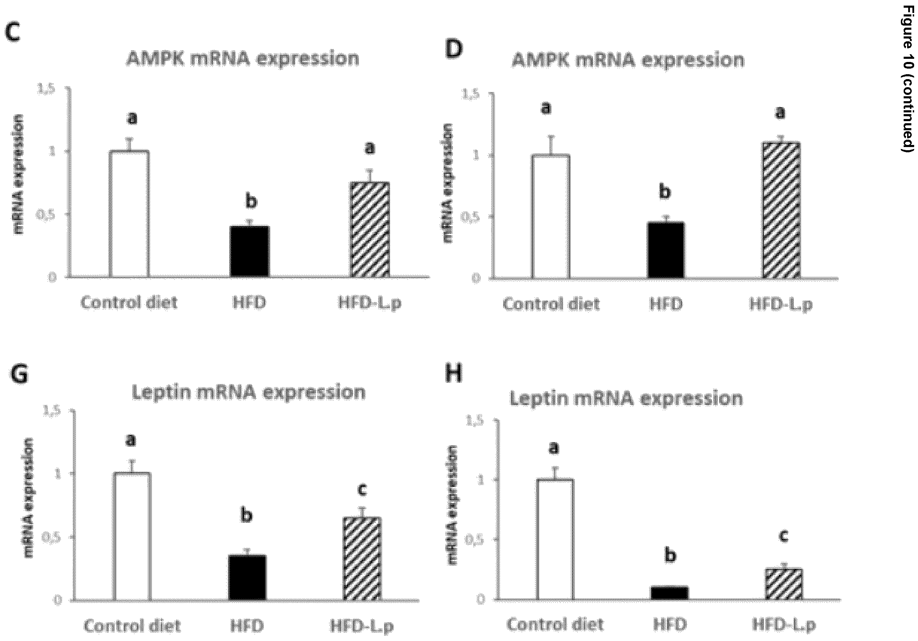

**Figure 10**. Effects of *L*. *paracasei* subsp. *tolerans* (CECT accession N° 9458) on metabolic-related marker gene expression in epididymal adipose tissue and liver. *L. paracasei* administration to mice increased the levels of A) *Glut4,* B) *Peroxisome proliferator-activated receptor (Ppar)-α* and C) *AMP-activated protein kinase (Ampk)* in epididymal fat and D) liver. It also increased the expression of E) adiponectin, while reducing the expression of F) leptin and in adipose tissue. Higher gene expression levels on leptin receptor with the bacterial strain administration were also found in G) epididymal fat and H) liver of the mice.

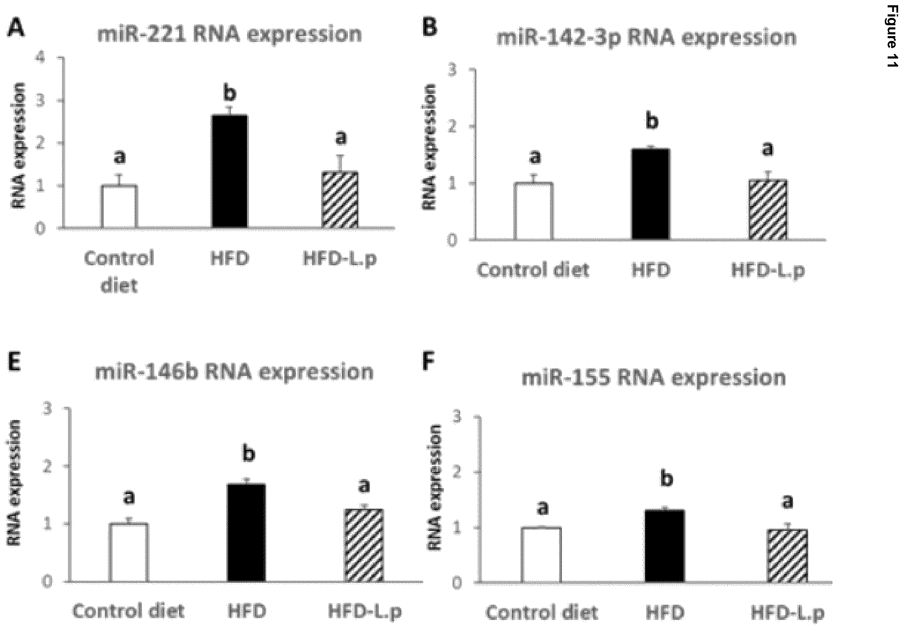

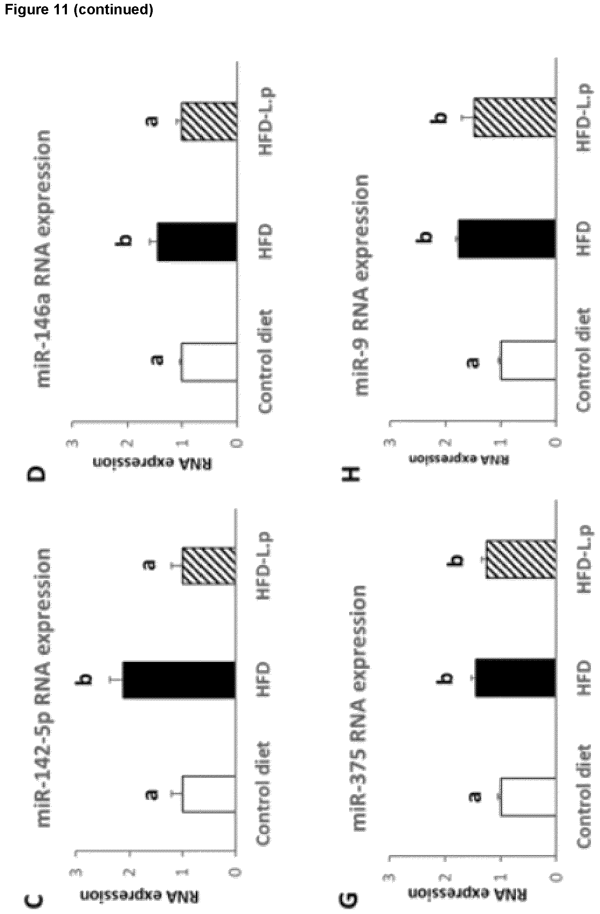

**Figure 11**. Effects of *L*. *paracasei* subsp. *tolerans* (CECT accession N° 9458) on inflammatory-related mi-RNA expression in epididymal adipose tissue. Data are expressed as means ± SEM (n = 10). Groups with different letter statistically differ (P < 0.05).

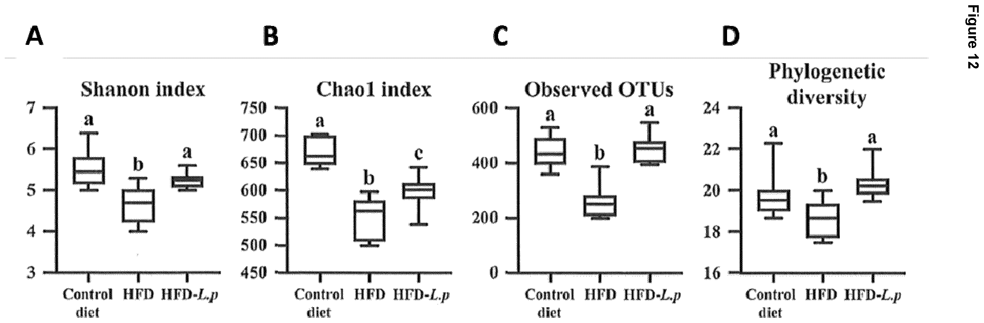

**Figure 12**. Impact of *L. paracasei* subsp. *tolerans* (CECT accession N° 9458) administration on microbiome diversity indicated by A) Shannon index, B) Chao1 index, C) Observed OTUs and D) Phylogenetic diversity. In all of them, *L. paracasei* administration increased microbial diversity. Data are expressed as means ±SEM. Groups with different letter statistically differ (P < 0.05).

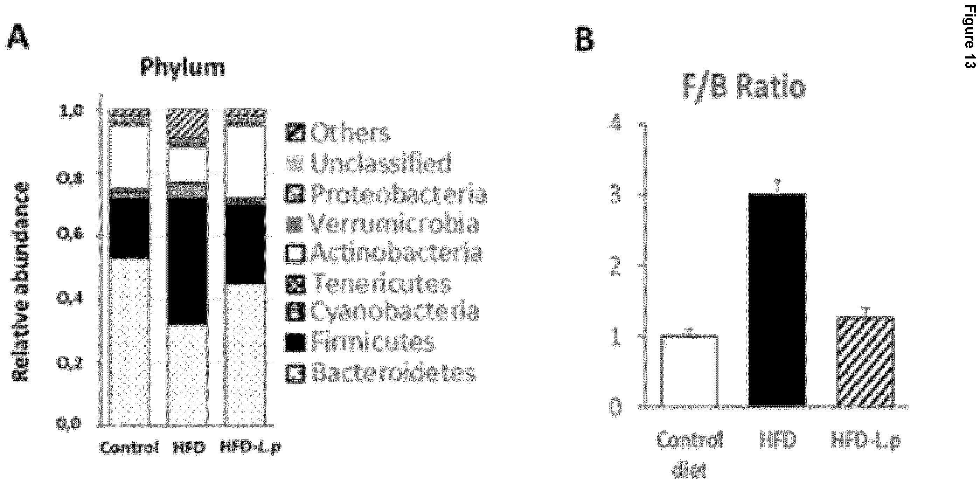

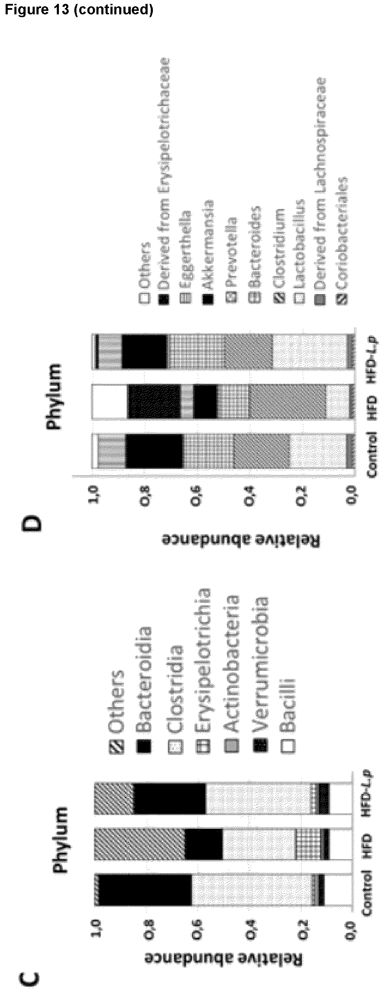

**Figure 13**. Impact of *L*. *paracasei* subsp. *tolerans* (CECT accession N° 9458) administration bacterial community at different levels A) phylum level, B) *Firmicutes*/*Bacteroidetes* ratio, C) class level, and D) genus level. Data are expressed as means ±SEM. Groups with different letter statistically

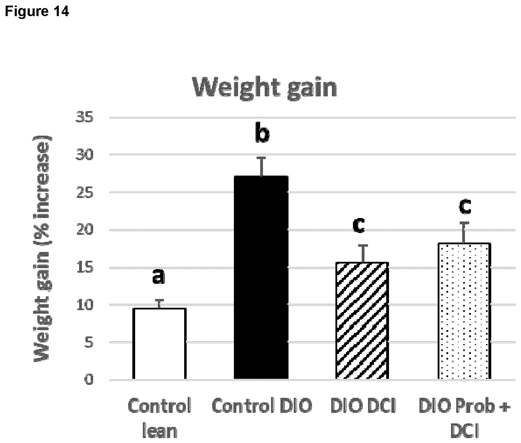

**Figure 14****.** Weight gain (% increase) after 5 weeks of treatment with Control diet (Lean), HFD diet (DIO), HFD diet + DCI (DIO DCI) and HFD diet +DCI+ *L. paracasei* subsp. *tolerans* (CECT accession N° 9458) (DIO Prob+DCI). Data are expressed as means ± SEM (n=10/group). Groups with different letter statistically differ (P< 0.05).

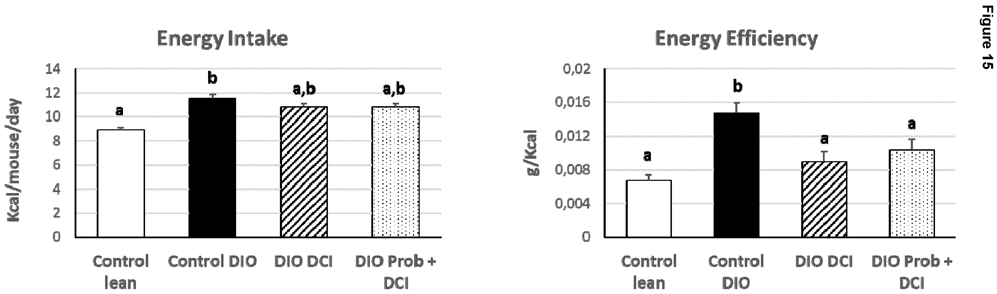

**Figure 15****.** A) Mean of energy intake per day during the 5 weeks of treatment with Control diet (Lean), HFD diet (DIO), HFD diet + DCI (DIO DCI) and HFD diet +DCI+ *L. paracasei* subsp. *tolerans* (CECT accession N° 9458) (DIO Prob+DCI). Data are expressed as means (Kcal/mourse/day) ± SEM (n=10). B) Feed efficiency during the 5 weeks of treatment with Control diet (Lean), HFD diet (DIO), HFD diet + DCI (DIO DCI) and HFD diet +DCI+ *L*. *paracasei* subsp. *tolerans* (CECT accession N° 9458) (DIO Prob+DCI). Data are expressed as means (g/Kcal) ± SEM (n=10/group). Groups with different letter statistically differ (P< 0.05).

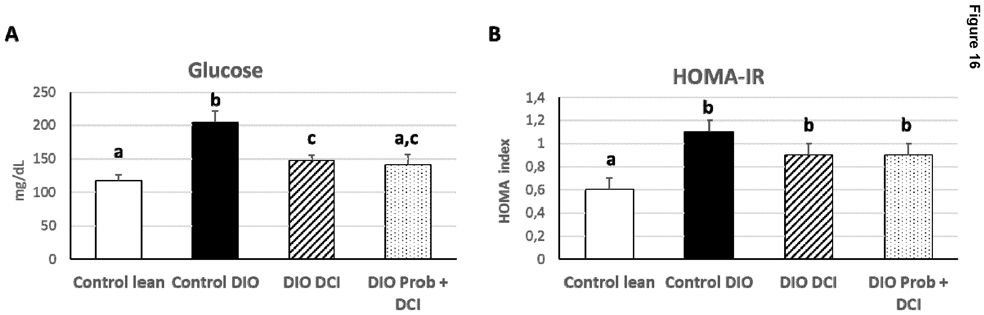

**Figure 16****.** Basal Glucose (A) and HOMA-IR index (B) after 5 weeks of treatment with Control diet (Lean), HFD diet (DIO), HFD diet + DCI (DIO DCI) and HFD diet +DCI+ *L. paracasei* subsp. *tolerans* (CECT accession N° 9458) (DIO Prob+DCI)Data are expressed as means ± SEM (n=10/group). Groups with different letter statistically differ (P< 0.05).

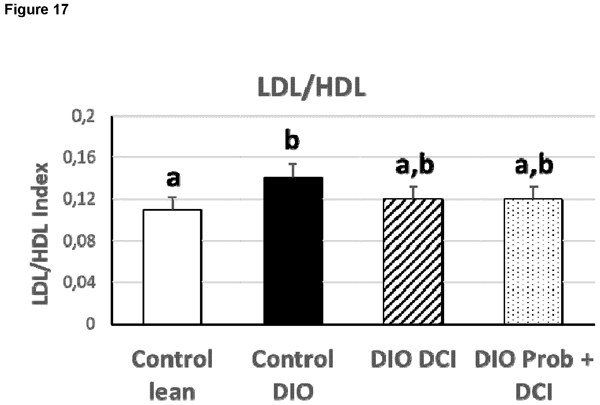

**Figure 17****.** LDL/HDL ratio after 5 weeks of treatment with Control diet (Lean), HFD diet (DIO), HFD diet + DCI (DIO DCI) and HFD diet +DCI+ *L. paracasei* subsp. *tolerans* (CECT accession N° 9458) (DIO Prob+DCI). Data are expressed as means ± SEM (n=10/group). Groups with different letter statistically differ (P< 0.05).

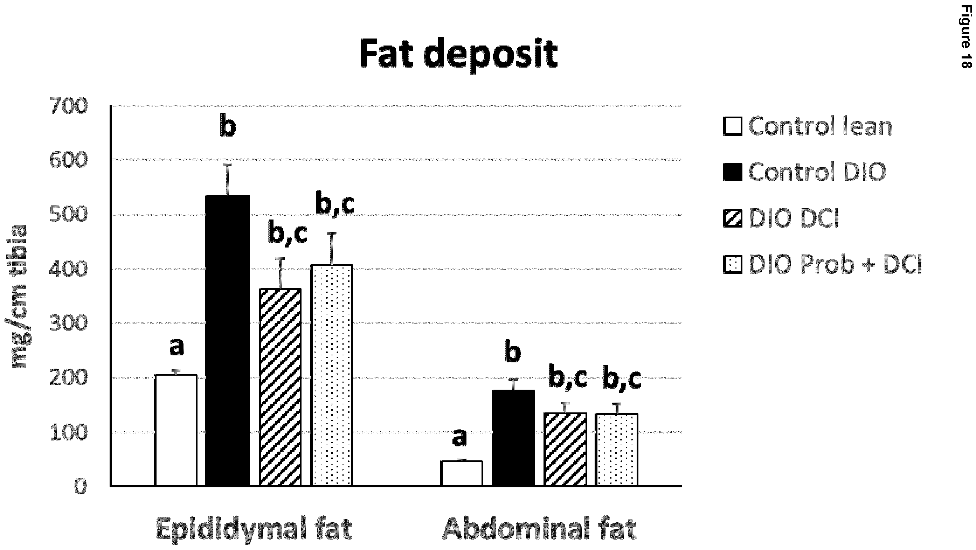

**Figure 18****.** Abdominal and epididymal fat after 5 weeks of treatment with Control diet (Lean), HFD diet (DIO), HFD diet + DCI (DIO DCI) and HFD diet +DCI+ *L. paracasei* subsp. *tolerans* (CECT accession N° 9458) (DIO Prob+DCI). Data are expressed as means ± SEM (n=10/group). Groups with different letter statistically differ (P< 0.05).

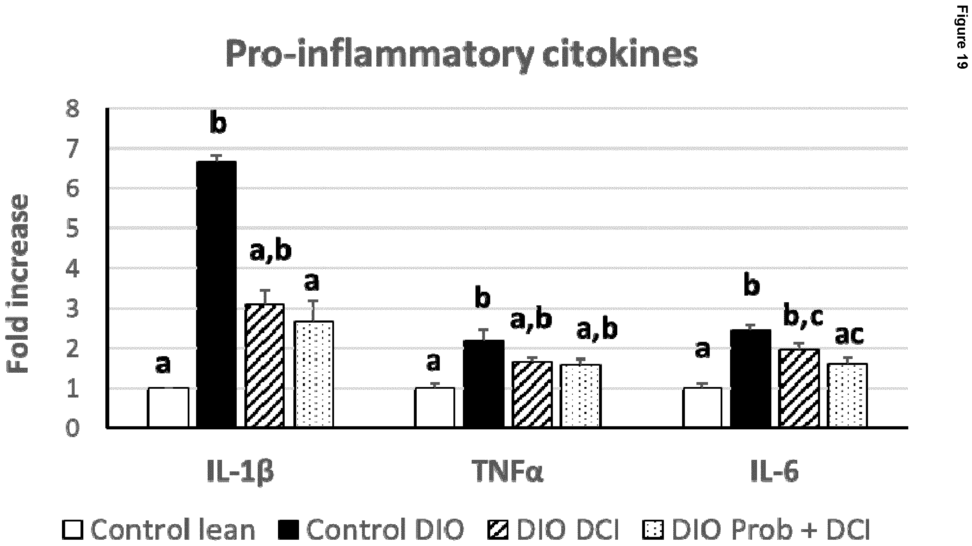

**Figure 19****.** Fold increase in level of mRNA expression of the pro-inflammatory cytokines IL-1β, TNFα and IL-6 in adipose tissue after 5 weeks of treatment with Control diet (Lean), HFD diet (DIO), HFD diet + DCI (DIO DCI) and HFD diet +DCI+ *L. paracasei* subsp. *tolerans* (CECT accession N° 9458) (DIO Prob+DCI). Data are expressed as means ± SEM (n=10/group). Groups with different letter statistically differ (P< 0.05).

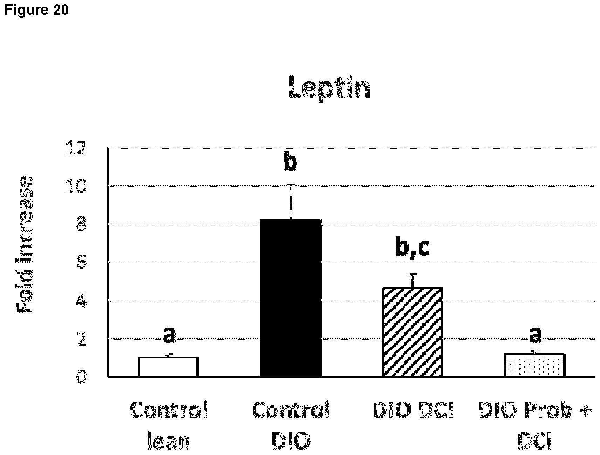

**Figure 20****.** Fold increase in level of mRNA expression of leptin in adipose tissue after 5 weeks of treatment with Control diet (Lean), HFD diet (DIO), HFD diet + DCI (DIO DCI) and HFD diet +DCI+ *L. paracasei* subsp. *tolerans* (CECT accession N° 9458) (DIO Prob+DCI). Data are expressed as means ± SEM (n=10/group). Groups with different letter statistically differ (P< 0.05).

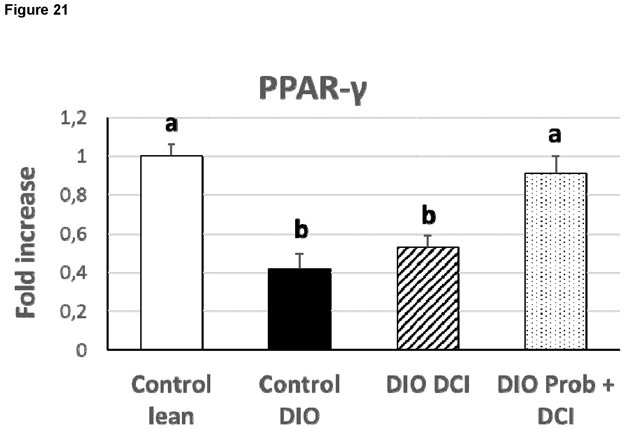

**Figure 21****.** Fold increase in level of mRNA expression of PPARγ in adipose tissue after 5 weeks of treatment with Control diet (Lean), HFD diet (DIO), HFD diet + DCI (DIO DCI) and HFD diet +DCI+ *L. paracasei* subsp. *tolerans* (CECT accession N° 9458) (DIO Prob+DCI). Data are expressed as means ± SEM (n=10/group). Groups with different letter statistically differ (P< 0.05).

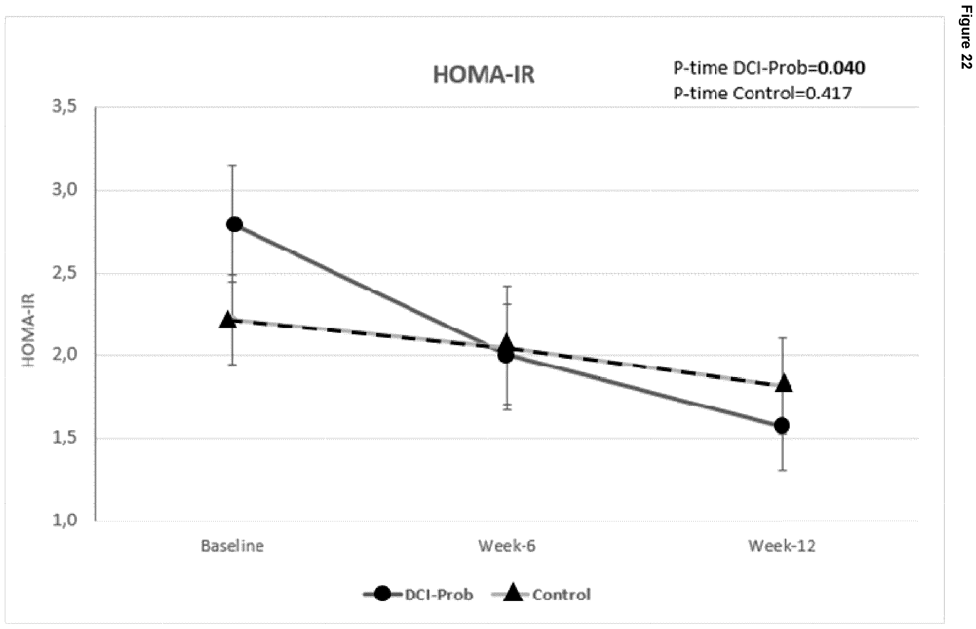

**Figure 22****:** HOMA-Index of the subjects consuming the placebo or the DCI-Prob at baseline, 6-weeks and 12-weeks. Mean values are represented with points (light grey for Control Group and dark grey for DCI-Prob group), whereas SE is represented by vertical bars. P-time for each group was calculated by linear mixed model adjusted by age, study site, smoking, drinking habits, physical activity, diabetes antecedents and BMI.

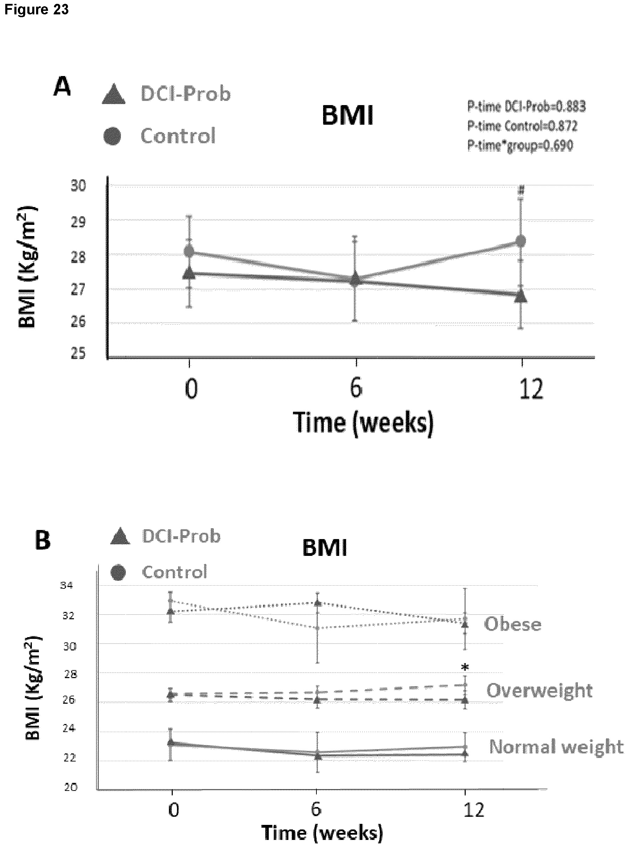

**Figure 23****:** A) BMI of the subjects consuming the placebo or the DCI-Prob at baseline, 6-weeks and 12-weeks. Mean values are represented with grey circles (Control Group) and grey triangles (DCI-Prob group), whereas SE is represented by vertical bars. B) BMI of the subjects consuming placebo or DCI-Prob classified by normal weigh (BMI<24.9), overweight (BMI 25-29.9) and obese (BMI>30). Mean values are represented with grey circles (Control Group) and grey triangles (DCI-Prob group), whereas SE is represented by vertical bars. * p value time-group= 0,041

### DETAILED DESCRIPTION OF THE INVENTION

The authors of the invention disclose herewith a new bacterial strain having therapeutic properties for the treatment of insulin resistance syndrome and associated conditions as well as infertility, gestational diabetes, obesity and proinflammatory states.

Unless otherwise defined, all technical terms used herein have the same meaning as commonly understood by one of ordinary skill in the art to which this invention belongs. The definitions provided herewith and in every other aspect of the invention are equally applicable to the whole invention.

### Bacterial strain

In a first aspect the invention relates to a bacterial strain of *Lacticaseibacillus paracasei* subsp. *tolerans.* Preferably, the bacterial strain is a strain of *Lacticaseibacillus paracasei* subsp. *tolerans* deposited under the terms of the Budapest Treaty on October 24^{th}, 2017 under accession number CECT 9458 in the Colección Española de Cultivos Tipo (CECT), University of Valencia, Parc Cientific UV Edificio 3 CUE, Catedrático Agustin Escardino, 9, 46980 Paterna (Valencia) SPAIN, or a variant thereof. Optionally, the bacterial strain is a probiotic strain.

The bacterial strain *Lacticaseibacillus paracasei* subsp. *tolerans* deposited in the CECT with accession number CECT 9458 is a bacterium of genus *Lacticaseibacillus,* species *L. paracasei,* and subspecies *tolerans.*

The Colección Española de Cultivos Tipo is also called the Spanish Type Culture Collection or CECT, as used herein these terms are synonymous.

The terms "CECT 9458" and "9458" are used herein synonymously to refer to the same accession number.

As used herein, the terms subspecies, subsp. and ssp. are synonymous; subsp. and ssp. are abbreviations for subspecies.

The term "strain", as used herein, refers to a genetic variant or subtype of a microorganism species, preferably a bacterial species. The term "probiotic strain", as used herein, refers to a live strain of a microorganism, preferably a bacterium, which, when administered in adequate amounts, confers a health benefit on the host or subject. Hosts or subjects suitable in the context of the invention includes any mammal, preferably primates, such as human beings and chimpanzees, pigs, horses, cows, goats, ewes, dogs, cats, rats and mice; more preferably human beings. Other suitable hosts include birds, preferably chickens, ducks, geese, swans, pheasants, pigeons, doves, and ostriches.

The strain of *Lacticaseibacillus paracasei* subsp. *tolerans* deposited in the Colección Española de Cultivos Tipo (CECT) with accession number CECT 9458 is also described herein by synonymous terms: *Lacticaseibacillus paracasei* subsp. *tolerans* (CECT 9458), *Lacticaseibacillus paracasei* subsp. *tolerans* ALE6, *Lacticaseibacillus paracasei* subsp. *tolerans* ALE6 (CECT 9458), ALE6, CECT 9458, and DES-0166. The terms "CECT 9458" and "9458" are used herein synonymously to refer to the same accession number.

The present invention contemplates a bacterial strain selected from:
*Lacticaseibacillus paracasei* subsp. *tolerans,* for example the specific *Lacticaseibacillus paracasei* subsp. *tolerans* strain deposited in the Colección Española de Cultivos Tipo (CECT) with accession number CECT 9458, or a variant thereof.

Thus the invention also relates to other strains of *Lacticaseibacillus paracasei* subsp. *tolerans, and* variants of the *Lacticaseibacillus paracasei* subsp. *tolerans* strain deposited in the CECT with accession number CECT 9458. As used herein, the terms "variant" or "mutant" of a strain refer to any naturally-occurring or specifically developed strain obtained from the reference strain *Lacticaseibacillus paracasei* subsp. *tolerans,* mainly by mutation, that maintains the properties of the reference strain. Thus, in the present case, the invention relates to variants of the reference strain in which one or more of the following properties associated to the CECT 9458 *Lacticaseibacillus paracasei* subsp. *tolerans* strain is preserved:
- Promote egg-laying of the age-1 *C. elegans* strain.
- Prevent weight gain in a diet-induced obesity model.
- Ability to reduce glucose spikes after ingestion in a diet-induced obesity model.
- Ability to cause a decrease in insulin resistance or an increase of insulin sensitivity in a diet-induced obesity model.
- Capacity to prevent LDL / HDL ratio increment in a diet-induced obesity model.
- Ability to promote a reduction in the content of abdominal and epididymal adipose tissue in a model of obesity induced by diet
- Ability to prevent the increase of inflammatory markers in a diet-induced obesity model.
- Ability to prevent the increase of leptin in a diet-induced obesity model.
- Ability to prevent AMPK, GLUT-4, PPAR-γ and adiponektin reduction in a diet-induced obesity model.
- Ability to prevent the increase of intestinal barrier function markers in a diet-induced obesity model.

As used herein, the expression "variant in which a property is preserved" is understood as being a variant that, while not necessarily showing an activity of 100% of the activity of the CECT 9458 *Lacticaseibacillus paracasei* subsp. *tolerans* strain from which it derives, the property is substantially preserved so that, when the property is measured using proper in vitro or in vivo quantitative assays, the variant shows at least 99%, at least 98%, at least 97%, at least 96%, at least 95%, at least 94%, at least 93%, at least 92%, at least 91%, at least 90%, at least 80%, at least 70%, at least 60%, at least 50% of the activity of the CECT 9458 *Lacticaseibacillus paracasei* subsp. *tolerans* strain from which it derives.

Thus, in a preferred embodiment, the variant is characterized in that its ability of promoting egg-laying of the age-1 *C. elegans* strain is of at least 99%, at least 98%, at least 97%, at least 96%, at least 95%, at least 94%, at least 93%, at least 92%, at least 91%, at least 90%, at least 80%, at least 70%, at least 60%, at least 50% of the activity of the CECT 9458 *Lacticaseibacillus paracasei* subsp. *tolerans* strain.

In another embodiment, the variant is characterized in that its ability of preventing weight gain in a diet-induced obesity model is strain is of at least 99%, at least 98%, at least 97%, at least 96%, at least 95%, at least 94%, at least 93%, at least 92%, at least 91%, at least 90%, at least 80%, at least 70%, at least 60%, at least 50% of the activity of the CECT 9458 *Lacticaseibacillus paracasei* subsp. *tolerans* strain.

In another embodiment, the variant is characterized in that its ability of reducing glucose spikes after ingestion in a diet-induced obesity model is of at least 99%, at least 98%, at least 97%, at least 96%, at least 95%, at least 94%, at least 93%, at least 92%, at least 91%, at least 90%, at least 80%, at least 70%, at least 60%, at least 50% of the activity of the CECT 9458 *Lacticaseibacillus paracasei* subsp. *tolerans* strain.

In another embodiment, the variant is characterized in that its ability induce a decrease in insulin resistance or an increase of insulin sensitivity in a diet-induced obesity model is of at least 99%, at least 98%, at least 97%, at least 96%, at least 95%, at least 94%, at least 93%, at least 92%, at least 91%, at least 90%, at least 80%, at least 70%, at least 60%, at least 50% of the activity of the CECT 9458 *Lacticaseibacillus paracasei* subsp. *tolerans* strain.

In another embodiment, the variant is characterized in that its ability to prevent LDL / HDL ratio increment in a diet-induced obesity model is of at least 99%, at least 98%, at least 97%, at least 96%, at least 95%, at least 94%, at least 93%, at least 92%, at least 91%, at least 90%, at least 80%, at least 70%, at least 60%, at least 50% of the activity of the CECT 9458 *Lacticaseibacillus paracasei* subsp. *tolerans* strain.

In another embodiment, the variant is characterized in that its ability to promote a reduction in the content of abdominal and epididymal adipose tissue in a model of obesity induced by diet is of at least 99%, at least 98%, at least 97%, at least 96%, at least 95%, at least 94%, at least 93%, at least 92%, at least 91%, at least 90%, at least 80%, at least 70%, at least 60%, at least 50% of the activity of the CECT 9458 *Lacticaseibacillus paracasei* subsp. *tolerans* strain.

In another embodiment, the variant is characterized in that its ability to prevent the increase of inflammatory markers in a diet-induced obesity model is of at least 99%, at least 98%, at least 97%, at least 96%, at least 95%, at least 94%, at least 93%, at least 92%, at least 91%, at least 90%, at least 80%, at least 70%, at least 60%, at least 50% of the activity of the CECT 9458 *Lacticaseibacillus paracasei* subsp. *tolerans* strain.

In another embodiment, the variant is characterized in that its ability to prevent the increase of leptin in a diet-induced obesity model model is of at least 99%, at least 98%, at least 97%, at least 96%, at least 95%, at least 94%, at least 93%, at least 92%, at least 91%, at least 90%, at least 80%, at least 70%, at least 60%, at least 50% of the activity of the CECT 9458 *Lacticaseibacillus paracasei* subsp. *tolerans* strain.

In another embodiment, the variant is characterized in that its ability to prevent AMPK, GLUT-4, PPAR-γ and adiponectine reduction in a diet-induced obesity model is of at least 99%, at least 98%, at least 97%, at least 96%, at least 95%, at least 94%, at least 93%, at least 92%, at least 91%, at least 90%, at least 80%, at least 70%, at least 60%, at least 50% of the activity of the CECT 9458 *Lacticaseibacillus paracasei* subsp. *tolerans* strain.

In another embodiment, the variant is characterized in that its ability to prevent the increase of intestinal barrier function markers in a diet-induced obesity model is of at least 99%, at least 98%, at least 97%, at least 96%, at least 95%, at least 94%, at least 93%, at least 92%, at least 91%, at least 90%, at least 80%, at least 70%, at least 60%, at least 50% of the activity of the CECT 9458 *Lacticaseibacillus paracasei* subsp. *tolerans* strain.

Variants may or may not have the same identifying biological characteristics of the specific strains exemplified herein, provided they share similar advantageous properties of the reference strain. For example, the 16S rRNA genes of a "variant" strain as contemplated herein may share about 85%, 86%, 87%, 88%, 89%, 90%, 91%, 92%, 93%, 94%, 95%, 96%, 97%, 98% or 99% sequence identity with a strain disclosed herein.

In another embodiment, a variant of the strain according to the present invention refers to any strain the genome of which hybridizes under stringent conditions with the genome of CECT 9458 *Lacticaseibacillus paracasei* subsp. *tolerans* strain. In general, a low stringency hybridization reaction is carried out at about 40 degrees centigrade in 10x SSC or a solution of equivalent ionic strength/temperature. A moderate stringency hybridization is typically performed at about 50 degrees centigrade in 6x SSC, and a high stringency hybridization reaction is generally performed at about 60 degrees centigrade in 1x SSC.

In another embodiment, the degree of relatedness between the variant and the parent strain is determined as the average nucleotide identity (ANI), which detects the DNA conservation of the core genome. In some embodiments, the ANI between the variant and the parent strain is of about 95%, about, 96%, about 97%, of about 98%, of about 99%, of about 99,1%, of about 99,5%, of about 99,6%, of about 99,7%, of about 99,8%, of about 99,9%, of about 99,99%, of about 99,999%, of about 99,9999%, of about 99,99999%, of about 99,999999% or more but less than 100%.

In another embodiment, the degree of relatedness between the variant and the parent strain is determined as the Tetranucleotide Signature Frequency Correlation Coefficient, which is based on oligonucleotide frequencies. In some embodiments, the Tetranucleotide Signature Frequency Correlation coefficient between the variant and the parent strain is of about 0,99, 0,999, 0,9999, 0,99999, 0,999999, 0,999999 or more but less than 1.

In another embodiment, the degree of relatedness between the variant and the parent strain is determined as the degree of similarity obtained when analyzing the genomes of the parent and of the variant strain by Pulsed-field gel electrophoresis (PFGE) using one or more restriction endonucleases. The degree of similarity obtained by PFGE can be measured by the Dice similarity coefficient. In some embodiments, the Dice similarity coefficient between the variant and the parent strain is of about 95%, about, 96%, about 97%, of about 98%, of about 99%, of about 99,1%, of about 99,5%, of about 99,6%, of about 99,7%, of about 99,8%, of about 99,9%, of about 99,99%, of about 99,999%, of about 99,9999%, of about 99,99999%, of about 99,999999% or more but less than 100%.

In another embodiment, a strain is considered a variant of a given parent strain when both strains have the same ribotype, as obtained using any of the methods known in the art an described, for instance, by Bouchet *et al.* (Bouchet, Huot and Goldstein. Molecular Genetic Basis of Ribotyping. Clin. Microbiol. Rev., 2008, 21:262-273).

In another embodiment, the degree of relatedness between the variant and the parent strain is the Pearson correlation coefficient obtained by comparing the genetic profiles of both strains obtained by repetitive extragenic palindromic element-based PCR (REP-PCR). In some embodiments, the Pearson correlation coefficient obtained by comparing the REP-PCR profiles of the variant and the parent strain is of about 0,99, 0,999, 0,9999, 0,99999, 0,999999, 0,999999 or more but less than 1.

In another embodiment, the degree of relatedness between the variant and the parent strain is the linkage distance obtained by comparing the genetic profiles of both strains obtained by Multilocus sequence typing (MLST). In some embodiments, the linkage distance obtained by MLST of the variant and the parent strain is of about 0,99, 0,999, 0,9999, 0,99999, 0,999999, 0,999999 or more but less than 1.

In a preferred embodiment, the variant and the parent strain are of the same genus. In a still more preferred embodiment, the variant and the parent strain are of the same species or subspecies.

In a particular embodiment the bacterial strain of the invention may be alive or inactivated.

As used herein, the term "inactivated" refers to a dead or inactivated cell of a microorganism which is no longer capable of forming a single colony on a plate having medium specific for said microorganism, and it also encompasses lysates, fractions or extracts of the microorganism. As used herein, "partially inactivated" specifies that at least some of the cells are inactivated, and "totally inactivated" specifies that substantially all of the cells are inactivated. The inactivation of a cell refers to a process of transforming a cell from viable to non-viable. The terms "viable", "alive" or "viability", as used herein, refer to the ability of a cell to maintain itself or recover its potentialities and survive until they are able to divide. Thus, a cell that is viable or alive indicates that the cell is able to survive and divide; conversely, a cell that is non-viable indicates that the cell is not able to survive and divide. It will be appreciated that non-viable cells include dead cells. Viability can be determined by a number of assays that are conventional in the art. These include cytolysis or membrane leakage assays, such as the lactate dehydrogenase assay, the propidium iodide assay, the trypan blue assay and the 7-aminoactinomycin D assay, as well as genomic and proteomic assays that test the activation of stress pathways using DNA microarrays and protein chips. Viability can also be determined by checking the absence of cells after their culture in an appropriate culture medium. Therefore, an inactivated CECT 9458 *Lacticaseibacillus paracasei* subsp. *tolerans* strain is different to the alive strain.

As a result of an inactivating step, the cells of the bacterial strain of the invention become non-viable, or even dead.

In a preferred embodiment the bacterial strain of the invention is in a totally inactivated form.

In another embodiment the bacterial strain of the invention has been inactivated by an inactivation process selected from the group consisting of thermal inactivation, microwave inactivation, pressure inactivation, acid inactivation, base inactivation, ethanol inactivation and peroxide inactivation.

The term "microwave inactivation", as used herein, refers to the inactivation of cells using radiofrequency waves, which are usually used at a frequency of 2450 MHz. Typically, the microwaves produced by a "home-type" microwave oven (2.45 GHz) completely inactivate bacterial cultures, mycobacteria, viruses, and G. stearothermophilus spores within 60 seconds to 5 minutes depending on the challenge organism.

The term "pressure inactivation", as used herein, refers to the inactivation of cells using pressure. Methods suitable for pressure inactivation are well-known in the art and include, without limitation, homogenization, for example, using a homogenizer or a French press. For example, cell inactivation may be achieved using a high pressure homogenizer at a pressure of 1,500-2,000 bar and 15-20 pulses/minute for 10 minutes.

The term "acid inactivation", as used herein, refers to the inactivation of cells by lowering the pH. This is normally achieved by adding an acid to the cells or the medium containing them and subsequently neutralizing the cells or the medium. Strong acids are preferred for the purposes of inactivating cells, and include, without limitation, hydrochloric acid (HCl), hydroiodic acid (HI), hydrobromic acid (HBr), perchloric acid (HClO₄), nitric acid (HNOs), and sulfuric acid (H₂SO₄). The strength of an acid refers to its ability or tendency to lose a proton. For example, inactivation may be achieved by adding H₂SO₄ to the cells until a concentration of 80 mM is reached, incubating the cells at 55°C for 4 h, and neutralizing by adding 10 M NaOH until the pH is 7.

Analogously, the term "base inactivation", as used herein, refers to the inactivation of cells using increasing the pH. This is normally achieved by adding a base to the cells or the medium containing them and subsequently neutralizing the cells or the medium. Strong bases are preferred for the purposes of inactivating cells, and include, without limitation, potassium hydroxide (KOH), barium hydroxide [Ba(OH)₂], cesium hydroxide (CsOH), sodium hydroxide (NaOH), strontium hydroxide [Sr(OH)₂], calcium hydroxide [Ca(OH)₂], lithium hydroxide (LiOH), and rubidium hydroxide (RbOH). The strength of a base refers to its ability to deprotonate an acid. For example, inactivation may be achieved by adding NaOH to the cells until a concentration of 80 mM is reached, incubating the cells at 55°C for 4 h, and neutralizing by adding 96% H2S04 until the pH is 7.

The term "alcohol inactivation", as used herein, refers to the inactivation of cells using alcohol. An alcohol is an organic hydroxyl compound with the -OH functional group bound to a saturated carbon atom, and includes ethanol, methanol, isopropanol, butanol; preferably ethanol. For example, cell inactivation may be achieved by making a 1:1 dilution of the cell culture with 96% ethanol, and incubating at 37°C for 1 h. Subsequently, the ethanol is eliminated by evaporation in a rotary evaporator or rotavap.

The term "peroxide inactivation", as used herein, refers to the inactivation of cells using peroxide. A peroxide is a compound containing an oxygen-oxygen single bond or the peroxide anion, 0₂²⁻, and includes hydrogen peroxide (H₂O₂), superoxides, dioxygenyls, ozones and ozonides. For example, cell inactivation may be achieved by incubating the cells in 1.5% (v/v) H₂0₂ at 37°C for 1 h. Subsequently, the H₂O₂ is eliminated by treating the cells with catalase.

The term "thermal inactivation" or "heat inactivation", as used herein, refers to the inactivation of cells using high temperatures. Usually, this is achieved by increasing the temperature of the cells or the medium containing them to over 50°C.

In a still preferred embodiment, the CECT 9458 *Lacticaseibacillus paracasei* subsp. *tolerans* strain of the invention is inactivated through thermal inactivation. Thermal inactivation can be achieved by different means, for example, without limitation, by incubation in a water bath or by autoclaving the bacterium. In an even more preferred embodiment, the thermal inactivation process to inactivate the *L. paracasei* of the invention comprises heating the strain at a temperature between 50°C and 150°C, between 60°C and 120°C, between 65°C and 100°C, between 70°C and 90°C, between 75°C and 85°C, and more preferably between 80°C and 85°C for a period between 15 minutes and 90 minutes, between 20 minutes and 60 minutes, between 25 and 50 minutes, between 30 and 45 minutes; more preferably in a water bath. In a preferred embodiment, the thermal treatment is applied during a period of 45 minutes, 40 minutes, 35 minutes and more preferably during 30 minutes. In a more preferred embodiment the thermal inactivation process comprises heating the bacterium in a water bath at a temperature between 80°C and 85°C for a period between 30 minutes and 45 minutes.

### Cultures, compositions, supernatants and pharmaceutical compositions according to the invention

In another aspect the invention relates to a biologically pure culture, or a composition, or a pharmaceutical product, or a feed or nutritional product, or a nutraceutical composition comprising a bacterial (optionally probiotic) strain of the invention.

The term "biologically pure culture", as used herein, refers to a culture in which the bacteria of the invention is found in a ratio of 90% or over, for example 95 % or over, 96% or over, 97% or over, 98% or over, 99% or over or 100%, compared to other organisms present in the culture. The term "culture", as used herein, refers to a population of the bacteria of the invention. A culture may comprise other elements than the bacteria of the invention, such as the culture medium or any other substance that could be added to the culture medium beneficial for the culture growth or maintenance. The term "culture medium" or "medium" is recognized in the art, and refers generally to any substance or preparation used for the cultivation of living cells. The term "medium", as used in reference to a cell culture, includes the components of the environment surrounding the cells. Media may be solid, liquid, gaseous or a mixture of phases and materials. Media include liquid growth media as well as liquid media that do not sustain cell growth. Media also include gelatinous media such as agar, agarose, gelatin and collagen matrices. Exemplary gaseous media include the gaseous phase that cells growing on a petri dish or other solid or semisolid support are exposed to. The term "medium" also refers to material that is intended for use in a cell culture, even if it has not yet been contacted with cells. In other words, a nutrient rich liquid prepared for bacterial culture is a medium. Similarly, a powder mixture that when mixed with water or other liquid becomes suitable for cell culture may be termed a "powdered medium". "Defined medium" refers to media that are made of chemically defined (usually purified) components. "Defined media" do not contain poorly characterized biological extracts such as yeast extract and beef broth. "Rich medium" includes media that are designed to support growth of most or all viable forms of a particular species. Rich media often include complex biological extracts. Any conventional culture medium appropriate for lactic acid bacteria or bifidobacteria culture known in the art can be used in the present invention, such as, for instance, MRS medium, HANK'S medium, APT medium, RCM medium, LM17 medium, BSM medium and Elliker medium.

"Composition", as used in the present invention relates to any composition of matter comprising the bacterial strain of the invention, e.g. a strain of *Lacticaseibacillus paracasei* subsp. *tolerans,* and/or the strain *Lacticaseibacillus paracasei* subsp. *tolerans* CECT 9458, or a variant thereof.

As used in the present invention, the expressions "pharmaceutical composition" or "pharmaceutical product" are used herein interchangeably and refer to a formulation which has been adapted to administer a predetermined dose of one or several therapeutically useful agents to a cell, a group of cells, an organ, a tissue or an animal in which a direct or indirect therapeutic effect of the composition is sought out. The pharmaceutical composition of the invention contains a pharmaceutical effective amount of a composition, which is understood herein as an amount capable of providing a therapeutic effect, and which can be determined by the person skilled in the art by commonly used means. In the particular case when the strain is formulated in compositions for oral administration, the concentration of microorganism(s) can be adjusted so as to correspond to doses (expressed in equivalent of microorganism) ranging from 5.10² to 10¹³ CFU/d and in particular from 10⁵ to 10¹⁰ CFU/d. The amount of the bacterial strain of the invention will vary depending upon the subject and the particular mode of administration. Methods to determine suitable dosages are well known in the art.

The compositions may be for human or animal usage in human and veterinary medicine and will typically comprise any one or more of a pharmaceutically acceptable diluent, carrier, or excipient. The term "carrier" refers to a diluent or excipient with which the active ingredient is administered. Acceptable carriers or diluents for therapeutic use are well known in the pharmaceutical art. The choice of pharmaceutical carrier, excipient or diluent can be selected with regard to the intended route of administration and standard pharmaceutical practice. For example, such pharmaceutical carriers can be sterile liquids, such as water and oils, including those of petroleum, animal, plant or synthetic origin, such as peanut oil, soybean oil, mineral oil, sesame oil and the like. Water or aqueous solutions of saline solution and aqueous dextrose and glycerol solutions, particularly for injectable solutions, are preferably used as carriers. The compositions may comprise as - or in addition to - the carrier, excipient or diluent any suitable binder(s), lubricant(s), suspending agent(s), coating agent(s), solubilizing agent(s). Preservatives, stabilizers, dyes and even flavoring agents may be provided in the pharmaceutical composition. Examples of preservatives include sodium benzoate, sorbic acid and esters of p-hydroxybenzoic acid. Antioxidants and suspending agents may also be used. There may be different composition/formulation requirements depending on the different delivery systems.

In a particular embodiment the composition is administered as part of a nutritional composition or nutraceutical composition comprising the composition for use according to the invention. The terms "nutritional composition", "feed" or "nutritional product" of the present invention relate to the food that beneficially affects one or more functions of the body, so as to provide better health and wellness. Accordingly, such a nutritional composition may be intended for the prevention and/or treatment of a disease or a disease causing factor. Therefore, the term "nutritional composition" of the present invention can be used as a synonym for functional food or foods for particular nutritional purposes, or medical food. A nutritional composition is similar to that of a conventional food and consumed as part of a normal diet appearance. Preferably, the feed or nutritional product comprises at least between 0.1 % and 99.9%, between 1 % and 99%, between 10% and 90%, between 20% and 80%, between 30% and 70%, between 40% and 60% of the strain of the invention. Non-limiting examples of suitable foodstuffs which can be used in the present invention are cereals, fermented cereal based products, other cereal based powders, clinical nutrition formula, bread, cakes or candies, animal feed formulations, semi- or synthetic diet formulations, infant formulae, clinical nutrition formulae, flours, bread, cakes, candies or chewing-gums.

In another embodiment, the bacterial strain of the invention is administered as part of a nutraceutical composition. By "nutraceutical", a word derived from nutrition and pharmaceutical, relates to a product made from a food, but which can be found in pill form, a powder and/or any other dosage forms not usually associated with food and having beneficial properties for the treatment and/or prevention of diseases.

In a particular embodiment the bacterial strain, or the biologically pure culture, composition, pharmaceutical product, feed, nutritional product, or nutraceutical composition is in a frozen, lyophilized or dried form, which can be obtained by any conventional method known in the art.

In a further aspect the bacterial (optionally probiotic) strain or the biologically pure culture, composition, pharmaceutical product, feed, nutritional product, or nutraceutical composition of the invention is used as a medicament.

### D-chiro-inositol

The inventors have unexpectedly found that a combination of D-chiro-inositol with a bacterial strain of *Lacticaseibacillus paracasei* subsp. *tolerans* is advantageous. Furthermore, a combination of D-chiro-inositol with the bacterial strain *Lacticaseibacillus paracasei* subsp. *tolerans* deposited in the CECT under Accession N° 9458, or a variant thereof, is particularly advantageous.

Therefore, the invention also relates to a combination of D-chiro-inositol with a bacterial strain of *Lacticaseibacillus paracasei* subsp. *tolerans,* optionally the bacterial strain *Lacticaseibacillus paracasei* subsp. *tolerans* deposited in the CECT under Accession N° 9458, or a variant thereof.

The biologically pure culture, composition, pharmaceutical product, feed, nutritional product, or nutraceutical composition of the invention; may further comprise D-chiro-inositol.

The bacterial strain for use as a medicament of the invention; may further comprises D-chiro-inositol. The bacterial strain for use in the treatment and/or prevention of any condition, state or disease disclosed in this application, may further comprise D-chiro-inositol.

Cosmetic or medical methods of the invention may comprise administering bacterial strain in combination with D-chiro-inositol.

As used herein, the terms D-chiro-inositol, D-chiroinositol and DCI are synonymous.

### Therapeutic uses

It is herewith disclosed that the bacterial (optionally probiotic) strain, or the biologically pure culture, composition, pharmaceutical product, feed, nutritional product, or nutraceutical composition of the invention can be used in the treatment and/or prevention of a variety of conditions, as explained below.

As used herein the terms "treat, " "treatment, " or "treatment of" refers to reducing the potential for a certain disease or disorder, reducing the occurrence of a certain disease or disorder, and/or a reduction in the severity of a certain disease or disorder, preferably, to an extent that the subject no longer suffers discomfort and/or altered function due to it. For example, "treating" can refer to the ability of a composition of the invention when administered to a subject, to prevent a certain disease or disorder from occurring and/or to cure or to alleviate a certain disease symptoms, signs, or causes. "Treating" also refers to mitigating or decreasing at least one clinical symptom and/or inhibition or delay in the progression of the condition and/or prevention or delay of the onset of a disease or illness. Thus, the terms "treat," "treating" or "treatment of" (or grammatically equivalent terms) refer to both prophylactic and therapeutic treatment regimes.

The present application provides methods and compositions generally providing a therapeutic benefit or desired clinical results. A therapeutic benefit is not necessarily a cure for a particular disease or disorder, but rather encompasses a result which most typically includes alleviation of the disease or disorder or increased survival, elimination of the disease or disorder, reduction or alleviation of a symptom associated with the disease or disorder, prevention or alleviation of a secondary disease, disorder or condition resulting from the occurrence of a primary disease or disorder, diminishment of extent of disease, stabilized (i.e., not worsening) state of disease, delay or slowing of disease progression, amelioration or palliation of the disease state, and remission (whether partial or total), whether detectable or undetectable and/or prevention of the disease or disorder. Treatment also means prolonging survival as compared to expected survival if not receiving the treatment.

The term "prevention", "preventing" or "prevent", as used herein, relates to the administration of a composition according to the invention or of a pharmaceutical composition according to the invention to a female subject who has not been diagnosed as possibly having polycystic ovary syndrome, or ovarian hyperstimulation syndrome at the time of administration, but who would normally be expected to develop said disease or be at increased risk for said disease. The prevention intends to avoid the appearance of said disease. The prevention can be complete (e.g. the total absence of a disease). The prevention can also be partial, such that for example the occurrence of a disease in a subject is less than that which would have occurred without the administration of the combination or composition of the present invention. Prevention also refers to reduced susceptibility to a clinical condition. The prevention also includes reducing the risk of suffering the disease.

### Infertility

The authors of the present invention have demonstrated that the oral intake of the strain of the invention is able to promote the egg-laying in the *age-1 C. elegans* strain, thus showing its efficacy in the treatment of insulin resistance infertility. Gene age-1 encodes the catalytic subunit of phosphatidylinositol-3-kinase (PI-3-kinase), a molecule that has been shown to act down-stream of the mammalian insulin. Mutation in this gene affects longevity, metabolism, and fertility.

Therefore, in another aspect the invention relates to the bacterial (optionally probiotic) strain, or the biologically pure culture, composition, pharmaceutical product, feed, nutritional product, or nutraceutical composition of the invention; for use in the treatment and/or prevention of infertility in a female subject.

"Infertility" as used herein, relates to the inability of a woman to reproduce by natural means. Particularly, is a disease of the reproductive system defined by the failure to achieve a clinical pregnancy after 12 months or more of regular unprotected sexual intercourse (and there is no other reason, such as breastfeeding or postpartum amenorrhoea). The infertility can be produced by several reasons. In a preferred embodiment, the female subject to be treated shows failure in embryo implantation.

The term "female subject" refers to a female mammal and include, but are not limited to the Order Rodentia, such as mice; Order Logomorpha, such as rabbits; more particularly the Order Carnivora, including Felines (cats) and Canines (dogs); even more particularly the Order Artiodactyla, Bovines (cows) and Suines (pigs); and the Order Perissodactyla, including Equines (horses); and most particularly the Order Primates, Ceboids and Simoids (monkeys) and Anthropoids (humans and apes). The mammals of preferred embodiments are humans.

In a preferred embodiment the infertility is associated with diabetes, and, more in particularly with type 2 diabetes, polycystic ovary syndrome and/or with insulin resistance syndrome.

The term "type 2 diabetes", as used in the present invention, relates to a disease characterized by an inappropriate increase in blood glucose levels, which generates chronic complications as it affects large and small vessels and nerves. The underlying disorder in this disease is the difficulty for insulin action (in the form of a loss of tissue sensitivity to this hormone), which is called insulin resistance, and an inadequate secretion of insulin by the cells responsible for their production in the pancreas. In addition to increasing glucose concentration, faulty insulin action frequently translates into an increase in cholesterol and/or triglyceride levels.

"Polycystic ovary syndrome", as used herein relates to a common health problem caused by an imbalance of reproductive hormones linked in more than 75% of the cases to insulin resistance. Signs and symptoms of PCOS include irregular or no menstrual periods, heavy periods, excess body and facial hair, acne, pelvic pain, difficulty getting pregnant, and patches of thick, darker, velvety skin. Associated conditions include type 2 diabetes, obesity, obstructive sleep apnea, heart disease, mood disorders, and endometrial cancer.

In a preferred embodiment, the female subject is in a reproductive age, between menarche and menopause. In another preferred embodiment, the female subject is treated for ovarian stimulation and oocyte production. In another preferred embodiment, the female subject is a human female subject. In another preferred embodiment, the human female subject shows a BMI below 30 kg/m2, therefore, the female subject does not show obesity. In another preferred embodiment, the female subject is treated with folic acid.

In another preferred embodiment, the female subject is subjected to an ovarian stimulation treatment after the administration of the composition according to the method of the invention.

"Ovary stimulation treatment", as used herein relates to the use of drugs to stimulate the growth of one or more follicles. Different drugs can be used, such as Clomiphene Citrate, Gonadotropins (FSH and LH) or Human Chorionic Gonadotropins (hCG). In a preferred embodiment, the ovary stimulation treatment is gonadotropin-releasing hormone antagonist and FSH.

The term "insulin resistance", as used in the present invention, relates to a disorder wherein the cells do not respond correctly to insulin. As a result, the body produces more insulin in response to high blood glucose levels. Patients with insulin resistance frequently display high glucose levels and high circulating insulin levels. Insulin resistance is frequently linked to obesity, hypertension, and hyperlipidemia. Additionally, insulin resistance frequently appears in patients with type 2 diabetes.

### Polycystic Ovary Syndrome

In another aspect, the invention relates to the bacterial (optionally probiotic) strain, or the biologically pure culture, composition, pharmaceutical product, feed, nutritional product, or nutraceutical composition of the invention; for use in the treatment and/or prevention of polycystic ovary syndrome.

The term "polycystic ovary syndrome" (PCOS) has been described previously within the context of infertility therapeutic uses of the bacterial strain and the compositions of the invention, and applies equally to the present case.

### Gestational diabetes

In another aspect, the invention relates to the bacterial (optionally probiotic) strain, or the biologically pure culture, composition, pharmaceutical product, feed, nutritional product, or nutraceutical composition of the invention; for use in the treatment and/or prevention of gestational diabetes.

The term "gestational diabetes" refers to a condition in which a woman without diabetes develops high blood sugar levels during pregnancy. Gestational diabetes is caused by not enough insulin in the setting of insulin resistance. Risk factors include being overweight, previously having gestational diabetes, a family history of type 2 diabetes, and having polycystic ovarian syndrome.

### Insulin resistance syndrome

It is shown in the examples of the present application that the intake of the bacterial strain of the invention results in the amelioration of the pathologies associated with insulin resistance, such as the reduction of glucose spikes after ingestion and the increase of insulin sensitivity as well as the prevention of LDL, HDL cholesterol and LDL / HDL ratio increment in a diet-induced obesity model. In addition, the authors of the invention have shown that the strain of the invention is able to promote a reduction in the content of abdominal and epididymal adipose tissue, and to modulate obesity-related inflammatory markers in a diet-induced obesity model.

Accordingly, in another aspect the invention relates to the bacterial (optionally probiotic) strain, or the biologically pure culture, composition, pharmaceutical product, feed, nutritional product, or nutraceutical composition of the invention; for use in the treatment and/or prevention of insulin resistance syndrome and related conditions selected from type 2 diabetes, obesity, heart disease and high blood pressure.

The term "insulin resistance" has already been defined within the context of the therapeutic use of the bacterial strain or compositions of the invention in the treatment of infertility, and applies equally to this case.

"Insulin Resistance Syndrome" as defined herein means the concomitant existence in a subject of two or more of: dyslipidemia, hypertension, type 2 diabetes mellitus or impaired glucose tolerance (IGT) or a family history of type 2 diabetes mellitus, hyperuricaemia and/or gout, a pro-coagulant state, atherosclerosis, truncal obesity. A family history of type 2 diabetes mellitus as defined herein means having a first degree relation, sibling, parent or grandparent with type 2 diabetes mellitus. At the center of the Insulin Resistance Syndrome, also known as "Syndrome X" and "Metabolic Syndrome" in the biomedical literature is the common feature of tissue resistance to the action of insulin. This impaired biological response to insulin can be manifested in both the metabolic and vascular effects of insulin

The term "dyslipemia", as used in the present invention, relates to any pathological condition characterized by a disorder in the lipid metabolism, with a consequent disorder in lipid concentration (cholesterol, triglycerides and such like) and lipoproteins (high density lipoproteins) in the blood. Dyslipemias that can be treated with the methods of the present invention include, without limitation, hypercholesterolemia, hypertriglyceridemia, hyperlipoproteinemia of type I, Ila, IIb, III, IV, V, hyperchylomicronemia, combined hyperlipidemia, etc.

The term "obesity", as used in the present invention, relates to the definition of obesity provided by the WHO based on the body mass index (BMI), which consists of the ratio between the weight of a person (in kg) and the square of their height in meters. According to this criterion, a BMI lower than 18.5 kg/m2 is considered as insufficient weight or thinness, a BMI of 18.5-24.9 kg/m2 is considered a normal weight, a BMI of 25.0-29.9 kg/m2 is considered grade 1 of overweight, a BMI of 30.0-39.0 kg/m2 is considered a grade 2 of overweight and a BMI greater than or equal to 40.0 kg/m2 is considered morbid obesity. Alternatively, there are alternative methods for defining an individual's degree of obesity, such as the diameter of the waist measured at the midpoint between the lower limit of the ribs and the upper limit of the pelvis (in cm), the thickness of skin folds, and bioimpedance, based on the principle that a lean mass transmits electricity better than a fatty mass.

The term "type 2 diabetes", as used in the present invention, relates to a disease characterized by an inappropriate increase in blood glucose levels, which generates chronic complications as it affects large and small vessels and nerves. The underlying disorder in this disease is the difficulty for insulin action (in the form of a loss of tissue sensitivity to this hormone), which is called insulin resistance, and an inadequate secretion of insulin by the cells responsible for their production in the pancreas. In addition to increasing glucose concentration, faulty insulin action frequently translates into an increase in cholesterol and/or triglyceride levels.

The term "high blood pressure" or "arterial hypertension" relates to a situation in which the systolic, diastolic blood pressure or both are constantly or repeatedly above the normal ranges established according to the sex and age of the patient.

### Inflammation

The authors of the present invention have shown in the examples of the present application that the oral intake of the strain of the invention is able to prevent the increase of inflammatory markers in a diet-induced obesity model.

Thus, in another aspect the invention relates to the bacterial (optionally probiotic) strain, or the biologically pure culture, composition, pharmaceutical product, feed, nutritional product, or nutraceutical composition of the invention; for use in the treatment and/or prevention of proinflammatory state or condition.

The term "proinflammatory" has already been described in relation to infertility-associated conditions. Proinflammatory is used herein to refer to any condition, state or disease able to promote or produce inflammation. It is usually characterized by excessive chronic production of inflammatory mediators and cytokines. An inflammatory cytokine is a type of cytokine a (signaling molecule) that is secreted from immune cells and certain other cell types that promotes inflammation. Inflammatory cytokines are predominantly produced by T helper cells (Ths) and macrophages and are involved in the upregulation of inflammatory reactions. Inflammatory cytokines include, without limitation interleukin-1 (IL-1), IL-12, and IL-18, tumor necrosis factor (TNF), interferon gamma (IFN-gamma), and granulocyte-macrophage colony stimulating factor. Non-limitative examples of inflammatory mediators include Cell-surface CD14, Free radical generation, IL-1β, IL-2, IL-6, IL-8, IL-15, IFN-y, Leukemia inhibitory factor, Monocyte chemoattractant protein-1, Monocyte chemoattractant protein-2, Neopterin, Neutrophil elastase, Phospholipase A2, Plasminogen activator inhibitor-1, Platelet activating factor, Prostacyclins, Prostaglandins, Protein kinase, Soluble adhesion molecules, Thromboxane, TNF-α, Tyrosine kinase and Vasoactive neuropeptides. Proinflammation is related with the development of many diseases such as autoimmune disease, allergies, neurological diseases, cancer, sepsis, metabolic disorders and cardiovascular diseases.

In a particular embodiment the proinflammatory state is associated with an obesity-related condition.

The term obesity has been define within the context of insulin resistance syndrome and applies equally in this case. Obesity-related conditions include, without limitation heart disease and stroke, high blood pressure, diabetes, cancer, osteoarthritis, sleep apnea, asthma, gout, and gallbladder disease and stones.

### Cosmetic methods

As mentioned above, it is proven in the examples of the present application that the oral intake of the bacterial strain of the invention helps to reduce obesity in a diet-induced obesity model. Therefore, in a further aspect the invention relates to a cosmetic method for the treatment of obesity comprising the administration to a subject of the bacterial (optionally probiotic) strain, or the biologically pure culture, composition, feed, nutritional product, or nutraceutical composition of the invention,

The cosmetic method of the invention consists essentially of or comprises the administration or the application of the bacterial strain or the biologically pure culture, or the composition, feed, nutritional product, or nutraceutical composition of the invention. "Cosmetic" as used herein is non-therapeutic. Such methods do not involve the treatment of the human or animal body by therapy.

As used herein, the term "cosmetic methods" relates to a method used to reduce the body mass index (BMI), which consists of the ratio between the weight of a person (in kg) and the square of their height in meters.

In the particular case when the strain is formulated in compositions for oral administration, the concentration of microorganism(s) can be adjusted so as to correspond to doses (expressed in equivalent of microorganism) ranging from 5.10² to 10¹³ CFU/d and in particular from 10⁵ to 10¹⁰ CFU/d.

### Further aspects of the invention

The invention will be described by way of the following examples which are to be considered as merely illustrative and not limitative of the scope of the invention.

### EXAMPLES

### EXAMPLE 1

### Identification and characterization of Lacticaseibacillus paracasei CECT 9458

### Objective

To study the morphological, genetic, and biochemical characteristics of *Lacticaseibacillus paracasei* CECT 9458, to carry out its taxonomic classification, and to describe possible differences with other microorganisms.

### Isolation

*L. paracasei* ALE6 was isolated on De Man, Rogosa and Sharpe (MRS) agar medium at pH 4.5; inoculated with a human breast milk sample from a healthy donor and incubated at 37 °C and anoxia for 48 h. Additionally, the same microorganism was subsequently isolated from other breast milk samples from 6 different donors.

*L. paracasei* ALE6 was deposited in the Spanish Type Culture Collection (CECT) under the Budapest treaty and with accession number 9458.

### Species and strain identification

### MALDI-TOF

Initial identification of *L. paracasei* ALE6 at species level was carried out using matrix-assisted laser desorption/ionization-time of flight (MALDI-TOF) spectrometry on a MALDI-TOF mass spectrometer such as a Bruker MALDI Biotyper^{®} instrument. For this purpose, several colonies obtained from MRS agar were subjected to analysis and comparison of the proteomic fingerprint with a library of reference mass spectra. The results of these analyses are shown in Table 1.

**Table 1. Species identification by MALDI-TOF spectrometry**

| Rank [quality]¹ | Organism (best match) | Score |
|---|---|---|
| 1 [++] | *L. paracasei* ssp *paracasei* DSM 20006 DSM | 2.208 |
| 2 [++] | *L. paracasei* ssp *paracasei* DSM 2649 DSM | 2.178 |
| 3 [++] | *L. paracasei* ssp *paracasei* DSM 20312 DSM | 2.144 |
| 4 [++] | *L. paracasei* ssp *paracasei* DSM 8741 DSM | 2.069 |
| 5 [++] | *L. paracasei* ssp *paracasei* DSM 20244 DSM | 2.062 |
| 6 [++] | *L. paracasei* ssp *paracasei* i DSM 5457 DSM | 2.049 |
| 7 [++] | *L. paracasei* ssp *paracasei* DSM 20008 DSM | 2.005 |
| 8 [+] | *L. paracasei* ssp *paracasei* DSM 46331 DSM | 1.977 |
| 9 [+] | *L. paracasei* ssp *tolerans* DSM 20258T DSM | 1.933 |
| 10 [+] | *L. paracasei* ssp *paracasei* DSM 8742 DSM | 1.862 |

| | | |
|---|---|---|
| ¹[+++]: Highly probable species identification (range: 2.300-3.000). [++]: Secure genus identification, probable species identification (range: 2.000-2.299). [+]: Probable genus identification (range: 1.700-1.999). [-]: Not reliable identification (range: 0.000-1.699). | | |

### 16S rRNA gene sequence similarity

The sequence of the 16S rRNA gene of *L. paracasei* ALE6 was obtained after PCR amplification and sequencing of the resulting DNA fragment and was subsequently confirmed by comparison with the genomic sequence of the microorganism.

The sequence of the 16S rRNA gene of the strain of *L. paracasei* deposited under accession number 9458 is shown in the sequence listing as SEQ ID NO: 1, and also shown below:

1                                ag agtttgatcc tggctcagga tgaacgctgg cggcgtgcct
  43 aatacatgca agtcgaacga gttctcgttg atgatcggtg cttgcaccga gattcaacat
 103 ggaacgagtg gcggacgggt gagtaacacg tgggtaacct gcccttaagt gggggataac
 163 atttggaaac agatgctaat accgcataga tccaagaacc gcatggttct tggctgaaag
 223 atggcgtaag ctatcgcttt tggatggacc cgcggcgtat tagctagttg gtgaggtaat
 283 ggctcaccaa ggcgatgata cgtagccgaa ctgagaggtt gatcggccac attgggactg
 343 agacacggcc caaactccta cgggaggcag cagtagggaa tcttccacaa tggacgcaag
 403 tctgatggag caacgccgcg tgagtgaaga aggctttcgg gtcgtaaaac tctgttgttg
 463 gagaagaatg gtcggcagag taactgttgt cggcgtgacg gtatccaacc agaaagccac
 523 ggctaactac gtgccagcag ccgcggtaat acgtaggtgg caagcgttat ccggatttat
 583 tgggcgtaaa gcgagcgcag gcggtttttt aagtctgatg tgaaagccct cggcttaacc
 643 gaggaagcgc atcggaaact gggaaacttg agtgcagaag aggacagtgg aactccatgt
 703 gtagcggtga aatgcgtaga tatatggaag aacaccagtg gcgaaggcgg ctgtctggtc
 763 tgtaactgac gctgaggctc gaaagcatgg gtagcgaaca ggattagata ccctggtagt
 823 ccatgccgta aacgatgaat gctaggtgtt ggagggtttc cgcccttcag tgccgcagct
 883 aacgcattaa gcattccgcc tggggagtac gaccgcaagg ttgaaactca aaggaattga
 943 cgggggcccg cacaagcggt ggagcatgtg gtttaattcg aagcaacgcg aagaacctta
1003 ccaggtcttg acatcttttg atcacctgag agatcaggtt tccccttcgg gggcaaaatg
1063 acaggtggtg catggttgtc gtcagctcgt gtcgtgagat gttgggttaa gtcccgcaac
1123 gagcgcaacc cttatgacta gttgccagca tttagttggg cactctagta agactgccgg

1183 tgacaaaccg gaggaaggtg gggatgacgt caaatcatca tgccccttat gacctgggct
1243 acacacgtgc tacaatggat ggtacaacga gttgcgagac cgcgaggtca agctaatctc
1303 ttaaagccat tctcagttcg gactgtaggc tgcaactcgc ctacacgaag tcggaatcgc
1363 tagtaatcgc ggatcagcac gccgcggtga atacgttccc gggccttgta cacaccgccc
1423 gtcacaccat gagagtttgt aacacccgaa gccggtggcg taaccctttt agggagcgag
1483 ccgtctaagg tgggacaaat gattagggtg aagtcgtaac aaggtagccg taggagaacc
1543 tgcggctgg

Comparison of the 16S sequence using the BLAST algorithm (Altschul *et al.* 1990) with prokaryotic ribosomal sequences available in the GenBank database confirmed the initial identification at the species level of *L. paracasei* ALE6, with identity percentages greater than 99% with other strains of the same species (Table 2, Fig. 0A).

**Table 2. Similarity results (BLAST) of the 16S rRNA gene of L. paracasei ALE6 with ribosomal sequences deposited in GenBank**

| **Description** | **Scientific Name** | **Total Score** | **Query** Cover | % **Identity** |
|---|---|---|---|---|
| *Lacticaseibacillus paracasei* strain R094 16S ribosomal RNA, partial sequence | *Lacticaseibacillus paracasei* | 2811 | 0.98 | 100 |
| *Lacticaseibacillus paracasei* strain NBRC 15889 16S ribosomal RNA, partial sequence | *Lacticaseibacillus paracasei* | 2758 | 0.96 | 99.93 |
| Lacticaseibacillus *paracasei* subsp. tolerans strain NBRC 15906 16S ribosomal RNA, partial sequence | *Lacticaseibacillus paracasei* subsp. *tolerans* | 2750 | 0.96 | 99.87 |
| Lacticaseibacillus *paracasei* subsp. tolerans strain NBRC 15906 16S ribosomal RNA, partial sequence | *Lacticaseibacillus paracasei* subsp. *tolerans* | 2750 | 0.96 | 99.87 |
| *Lacticaseibacillus paracasei* strain ATCC 25302 16S ribosomal RNA, partial sequence | *Lacticaseibacillus paracasei* | 2645 | 0.92 | 99.79 |
| *Lacticaseibacillus chiayiensis* strain BCRC 81062 16S ribosomal RNA, partial sequence | *Lacticaseibacillus chiayiensis* | 2772 | 0.99 | 99.22 |
| *Lacticaseibacillus zeae* strain RIA 482 16S ribosomal RNA,partial sequence | Lacticaseibacillus *zeae* | 2747 | 0.98 | 99.21 |
| *Lacticaseibacillus casei* DSM 20011 = JCM 1134 = ATCC 393 16S ribosomal RNA, partial sequence | *Lacticaseibacillus casei* | 2728 | 0.97 | 99.14 |
| *Lacticaseibacillus casei* strain NBRC 15883 16S ribosomal RNA, partial sequence | *Lacticaseibacillus casei* | 2693 | 0.96 | 99.13 |
| *Lacticaseibacillus casei* DSM 20011 = JCM 1134 = ATCC 393 16S ribosomal RNA, partial sequence | *Lacticaseibacillus casei* | 2732 | 0.98 | 99.02 |
| *Lacticaseibacillus rhamnosus* strain NBRC 3425 16S ribosomal RNA, | *Lacticaseibacillus rhamnosus* | 2667 | 0.96 | 98.86 |
| partial sequence *Lacticaseibacillus casei* strain BCRC10697 16S ribosomal RNA, partial sequence | *Lacticaseibacillus casei* | 2713 | 0.98 | 98.76 |
| *Lacticaseibacillus rhamnosus* strain JCM 1136 16S ribosomal RNA, partial sequence | *Lacticaseibacillus rhamnosus* | 2687 | 0.97 | 98.55 |
| *Lacticaseibacillus* minshuiensis strain 117-1 16S ribosomal RNA, partial sequence | *Lacticaseibacillus mingshuiensis* | 2357 | 0.9 | 97.07 |
| Lacticaseibacillus *yichunensis* strain 33-1 16S ribosomal RNA, partial sequence | *Lacticaseibacillus yichunensis* | 2348 | 0.89 | 97.06 |
| *Lacticaseibacillus brantae* DSM 23927 strain SL1108 16S ribosomal RNA, partial sequence | *Lacticaseibacillus brantae* DSM 23927 | 2477 | 0.98 | 95.84 |

### Species-specific qPCR

The identity of different *L. paracasei* ALE6 cultures was confirmed at the species level also by PCR, amplifying species-specific fragments using two different pairs of primers (Table 3). Amplification conditions were 3 minutes of initial denaturation at 95 °C, 30 cycles of amplification for 30 s at 95 °C (denaturation), 30 s at 55 °C (annealing), 1 min at 72 °C (extension), followed by a final extension cycle of 3 min at 72 °C. Amplicon sizes were checked by agarose gel electrophoresis (data not shown).

**Table 3. Primers used to amplify specific fragments of L. paracasei.**

| Primer | Sequence | SEQ ID NO | Target |
|---|---|---|---|
| 16 Berthier | GCTGGATCACCTCCTTTC | 2 | 16S-23S spacer region |
| 16 paracasei ITS | CGATGCGAATTTCTTTTTC | 3 | (Berthier and Ehrlich, 1998) |
| Y2 | CCCACTGCTGCCTCCCGT AGGAGT | 4 | 16S rRNA |
| Y2 paracas | CACCGAGATTCAACATGG | 5 | (Ward and Timmins, 1999) |

### Ribotyping

Strain identification of *L. paracasei* ALE6 was performed by ribotyping, simultaneously analyzing the strain mentioned above and 3 other microorganisms (Kerry microorganism collection) isolated from breast milk samples and belonging to the same species. This technique is one of the most powerful for differentiating microorganisms at strain level and is based on restriction enzyme digestion of genomic DNA (EcoRI in this case), electrophoresis of the digested DNA in agarose gels (0.8% w/v) and transfer and hybridization by Southern-blot. The digoxigenin-labelled (DIG-labelled) probe used hybridized with the ribosomal gene operon, so only the fragments coding for such rRNA were visualized and analyzed. The polymorphism in these highly conserved sequences generates a genetic fingerprint that may be unique for each bacterial strain.

The electrophoretic profile after DNA hybridization of the 4 microorganisms (Fig. 0B) showed clear differences between *L. paracasei* ALE6 and the other *L. paracasei* strains, so we could conclude that *L. paracasei* ALE6 is indeed a different strain from the other microorganisms from the same species.

### Morphological and biochemical characteristics

### Morphological description

*Lacticaseibacillus paracasei* ALE6 CECT 9458 is a gram-positive, homofermentative, non-spore forming, rod shaped microorganism.

### Culture conditions

The optimal growth conditions for *L. paracasei* ALE6 on solid medium are incubation at 37 °C for 48 h on MRS agar medium and in an anoxic atmosphere, such as in an anoxic atmosphere incubator, for example using AnaeroGen (Thermo Fisher Diagnostics). On the other hand, incubation in liquid medium using MRS broth and at 15, 37 and 45 °C without shaking allows obtaining the concentrations of viable bacteria, quantified by the plate count method on MRS agar, shown in Table 4.

**Table 4. L. paracasei ALE6 concentration (cfu/mL) cultured in MRS broth.**

| Temperature | Time | |
|---|---|---|
| | 24 h | 48 h |
| 15°C | 4.17E+06 | 6.33E+06 |
| 37°C | 7.33E+08 | 9.17E+08 |
| 45°C | 1.10E+03 | 1.30E+03 |

The results indicate that 37 °C is the temperature that allows the greatest growth of the microorganism (7.33E+08 cfu/mL at 24 h and 9.17E+08 at 48 h of incubation), while slow growth rates were observed at 15 °C. Finally, incubation at 45 °C does not allow the division of *L. paracasei* ALE6, observing a concentration of viable bacteria at 24 and 48 h that indicates the almost absence of growth of the microorganism.

### D/L-lactate production

*L. paracasei* ALE6 is a microorganism belonging to the group of lactic acid bacteria, which produce L-lactic acid and/or D-lactic acid as a product of carbohydrate fermentation. To evaluate the production of these enantiomers, the bacteria was cultured in MRS broth for 24 h and the concentration of L-lactate and D-lactate in the extracellular medium was quantified spectroscopically using the D-/L-Lactic acid, UV method kit (NZYtech) according to the manufacturer's instructions. The culture supernatants showed a D-lactate concentration of 2.29±0.17 mM (0.21±0.02 g/L) and an L-lactate concentration of 85.72±1.17 mM (7.72±0.11 g/L), and therefore a D/L ratio of 0.027.

### Short chain fatty acid production

The ability to produce acetic, propionic, and butyric acids was studied in fermentation supernatants of *L. paracasei* ALE6 cultured in MRS broth medium for 24, 48 and 72 h. The results of this analysis, carried out by gas chromatography using a flame ionization detector (GC-FID), revealed concentrations indistinguishable from the control in the case of acetic acid and below the detection limit of the method in the case of propionic acid. On the other hand, butyric acid could be quantified at concentrations of 92.7±2.21, 92.3±0.01 and 91.9±0.62 mg/L at 24, 48 and 72 h of growth respectively.

### Enzymatic activities

An anzyme activity test kit, such as the API ZYM system (Biomerieux) was used to detect 19 enzymatic activities that can be useful to identify and differentiate bacterial strains. The results obtained in *L. paracasei* ALE6, from 2 independent analyses, are shown in Table 5 together with the results of other strains of the same species obtained from BacDive (https://bacdive.dsmz.de/; Reimer *et al.* 2022).

**Table 5. Enzymatic activities**

| Enzymes | ALE6 | I | II | III | IV | V | VI |
|---|---|---|---|---|---|---|---|
| Alkaline | | | | | | | |
| phosphatase | - | + | - | + | + | + | +/- |
| Esterase (C4) | - | + | - | + | + | - | + |
| Esterase Lipase | | | | | | | |
| (C8) | - | + | - | + | + | + | + |
| Lipase (C14) | - | - | - | - | + | - | - |
| Leucine arylamidase | + | + | + | + | + | + | + |
| Valine arylamidase | + | + | + | + | + | + | + |
| Cystine arylamidase | + | + | - | + | + | + | + |
| Trypsin | - | - | - | - | - | - | - |
| alpha-Chymotrypsin | - | - | - | - | + | - | - |
| Acid phosphatase | - | + | + | + | + | + | + |
| Naphthol-AS-BI-phosphohydrolase | - | + | + | + | + | + | + |
| alpha-Galactosidase | - | - | - | - | - | - | - |
| beta-Galactosidase | - | - | + | + | + | + | +/- |
| beta-Glucuronidase | - | - | - | - | - | - | - |
| alpha-Glucosidase | + | + | - | + | + | + | + |
| beta-Glucosidase | - | + | - | + | + | + | +/- |
| N-acetyl-beta-glucosaminidase | - | - | - | + | + | - | + |
| alpha-Mannosidase | - | - | - | - | - | - | - |
| alpha-Fucosidase | - | - | - | - | - | - | - |

| | | | | | | | |
|---|---|---|---|---|---|---|---|
| +, positive; -, negative; +/-, inconclusive. I, *L.* paracasei ATCC25302; II, *L. paracasei* ATCC25599; III, *L. paracasei* CECT980; IV *L. paracasei* CIP102309; V, *L. paracasei* CIP110104; VI, *L. paracasei* ATCC334. | | | | | | | |

As can be seen, there is no common pattern regarding the presence or absence of enzymatic activities in all microorganisms of the species. There are therefore differences at the enzymatic level both in *L. paracasei* ALE6 and in the other strains, which can be considered as distinctive characteristics.

### Carbohydrate fermentation

*L. paracasei* ALE6 was tested for its carbohydrate fermenting capacity using a test kit such as the API 50CH system (Biomerieux), following the manufacturer's instructions. The results presented in Table 6, together with the information available for other strains of the *L. paracasei* species (Reimer et al. 2022), were obtained from two independent experiments in which 2 replicates were performed in each of them.

**Table 6. Carbohydrate fermentation pattern**

| Carbon source | ALE6 | I | II | III | IV | V | VI |
|---|---|---|---|---|---|---|---|
| Glycerol | - | - | - | - | - | - | - |
| Erythritol | - | - | - | - | - | - | - |
| D-Arabinose | - | - | - | - | - | - | - |
| L-Arabinose | - | - | - | - | - | - | - |
| D-Ribose | + | + | + | - | + | - | + |
| D-Xylose | - | - | - | - | - | - | - |
| L-Xylose | - | - | - | - | - | - | - |
| D-Adonitol | - | - | - | - | - | - | - |
| Methyl-beta D-xylopyranoside | - | - | - | - | - | - | - |
| D-Galactose | + | + | + | + | + | + | + |
| D-Glucose | + | + | + | + | + | + | + |
| D-Fructose | + | + | + | + | + | + | + |
| D-Mannose | + | + | + | + | + | + | + |
| L-Sorbose | + | + | - | - | + | - | - |
| L-Rhamnose | - | - | - | - | - | - | - |
| Dulcitol | - | - | - | - | - | - | - |
| Inositol | - | - | - | - | - | - | - |
| D-Mannitol | + | + | + | - | + | - | + |
| D-Sorbitol | + | + | - | - | - | - | + |
| Methyl-alpha D-mannopyranoside | - | - | - | - | - | - | - |
| Methyl-alpha D-glucopyranoside | - | - | - | - | +/- | - | - |
| N-Acetylclucosamine | + | + | + | + | + | + | + |
| Amygdalin | - | + | - | - | - | - | + |
| Arbutin | + | + | + | - | + | - | + |
| Esculin ferric citrate | + | + | + | - | + | - | + |
| Salilcin | + | + | + | - | + | - | + |
| D-Cellobiose | + | + | + | - | + | - | + |
| D-Maltose | + | + | +/- | - | +/- | - | + |
| D-Lactose | + | + | + | + | + | + | - |
| D-Melibiose | +/- | - | - | - | - | - | - |
| D-Saccharose | + | + | +/- | - | +/- | - | + |
| D-Trehalose | + | + | + | - | + | - | + |
| Inulin | + | + | - | - | - | - | - |
| D-Melezitose | + | + | + | - | + | - | + |
| D-Raffinose | +/- | - | - | - | - | - | - |
| Starch | - | - | - | - | - | - | - |
| Glycogen | - | - | - | - | - | - | - |
| Xylitol | - | - | - | - | - | - | - |
| Gentiobiose | + | + | +/- | - | - | - | + |
| D-Turanose | + | + | + | - | + | - | + |
| D-Lyxose | - | - | - | - | - | - | - |
| D-Tagatose | + | + | + | - | + | - | + |
| D-Fucose | - | - | - | - | - | - | - |
| L-Fucose | - | - | - | - | - | - | - |
| D-Arabitol | - | - | - | - | - | - | - |
| L-Arabitol | + | - | - | - | - | - | + |
| Gluconate | + | + | +/- | - | +/- | - | + |
| 2-Ketogluconate | - | - | - | - | - | - | - |
| 5-Ketogluconate | - | - | - | - | - | - | - |

| | | | | | | | |
|---|---|---|---|---|---|---|---|
| +, positive; -, negative; +/-, inconclusive. I, *L. paracasei* ATCC25598; II, *L. paracasei* ATCC25302; III, *L. paracasei* ATCC25599; IV *L. paracasei* ATCC335; V, *L. paracasei* DSM20012; VI, *L. paracasei* DSM20006. | | | | | | | |

As in the case of the presence of certain enzymatic activities, there is also no common pattern of carbohydrate fermenting capacity in the *L. paracasei* species. Although *L. paracasei* ALE6 has in many cases the same capacity to ferment different carbon sources as the other strains, there is no complete similarity, and it could be concluded that *L. paracasei* ALE6 has some distinctive characteristics at the metabolic level.

### Antibiotic susceptibility

The assays to determine the minimal inhibitory concentration (MIC) of antimicrobials were carried out by two methods: ((1) the broth dilution method following the procedure described in ISO 10932:2012 "Milk and dairy products - Determination of minimum inhibitory concentration (MIC) of antibiotics applicable to bifidobacteria and non-enterococcal lactic acid bacteria (LAB)" (International Standard Organization, 2012) and (2) using a test kit for determining MIC such as the eTest method (Biomerieux) following the manufacturer's instructions. Two broth dilution inhibition experiments were performed to study susceptibility to the panel of antibiotics recommended by the European Food Safety Authority (EFSA). In addition, five eTest assays were performed to confirm the results obtained by the broth dilution method and to include other antibiotics in the study. The mode of the results was used to calculate the MIC values, selecting the highest value when there were discrepancies between the results obtained by the two methods. The limit concentrations, as a reference to know whether *L. paracasei* ALE6 was susceptible or resistant to a particular antibiotic, were those established according to the EFSA guideline (Rychen *et al.,* 2018). In the case of antimicrobials for which there is no defined limit for *L. paracasei,* the range of ECOFF (Epidemiological cut-off values) collected for microorganisms of other species was used as a reference (www.eucast.org).

The growth *of L. paracasei* ALE6 was inhibited at antimicrobial concentrations equal to or lower than the limits established by EFSA (Table 7), so it can be considered susceptible to those antibiotics. On the other hand, the probiotic strain showed MIC values within the range of ECOFF proposed for other bacterial species, in the case of the antibiotics Azithromycin, Ciprofloxacin and Trimethoprim/Sulfamethoxazole (Table 7).

**Table 7. Minimal inhibitory concentration (mg/L)**

| Antimicrobial | Broth | eTest | MIC | Limit¹ | ECOFF² |
|---|---|---|---|---|---|
| Ampicillin | 2 | 0.25 | 2 | 4 | |
| Azithromycin | - | 0.125 | 0.125 | n.r. | 0.125-16 |
| Chloramphenicol | 4 | 2 | 4 | 4 | - |
| Ciprofloxacin | - | 1.5 | 1.5 | n.r. | 0.008-32 |
| Clindamycin | 0.016 | 0.23 | 0.23 | 4 | - |
| Erythromycin | 0.031 | 0.047 | 0.047 | 1 | - |
| Gentamicin | 4 | 4 | 4 | 32 | - |
| Kanamycin | NC | 64 | 64 | 64 | - |
| Streptomycin | 16 | 32 | 32 | 64 | - |
| Tetracycline | 1 | 0.5 | 1 | 4 | - |
| Trimethoprim/Sulfamethoxazole (1/19) | - | 0.094 | 0.094 | n.r. | 0.06-32 |

| | | | | | |
|---|---|---|---|---|---|
| ¹EFSA microbiological cut-off values, n.r. not required. ²Minimum and maximum epidemiological cut-off values described for other species and genera. NC, not conclusive | | | | | |

### Genome analysis

### Genome sequencing

The *L. paracasei* ALE6 genome was sequenced using next-generation sequencing technology such as the Illumina platform (BaseClear, The Netherlands) and annotated using a prokaryotic genome annotating software tool such as a pipeline based on Prokka (Seemann 2014). A summary of the statistics of the sequencing, assembly and annotation process is presented in Table 8.

**Table 8. Genome statistics**

| Total reads | Mapped | Avrg. Cover. | N° sequences | Avrg. G+C | Genome size (bp) | N° genes | N° tRNA | N° rRNA |
|---|---|---|---|---|---|---|---|---|
| 2,053,068 | 99.32% | 94.58% | 249 | 46.55% | 3,148,53 | 2,957 | 57 | 3 |

### Average Nucleotide Identity (ANI) and Digital DNA-DNA hybridization (DDH)

The available genomic sequence was used to study the similarity of *L. paracasei* ALE6 with other representatives of the species and *L. casei* (Table 9), by calculating Average Nucleotide Identity using a software tool such as ANI Calculator (https://www.ezbiocloud.net/tools/ani; Yoon *et al.* 2017).

**Table 9. Genomic similarity (ANI)**

| **Genome¹** | **Length (bp)** | **G+C (%)** | **ANI (%)** |
|---|---|---|---|
| *L. paracasei* ALE6 | 3,148,530 | 46.55 | - |
| *L. paracasei* subsp *paracasei* 8700:2 | 3,025,352 | 46.30 | 98.43 |
| *L. paracasei* subsp *tolerans* Lpl14 | 3,014,095 | 46.27 | 98.73 |
| *L. casei* ATCC 393 | 2,952,961 | 47.88 | 77.54 |

| | | | |
|---|---|---|---|
| ¹Genomic sequences of 8700:2, Lpl14 and ATCC 393 were obtained from GenBank. | | | |

Additionally, we carried out a DNA-DNA digital hybridization study using a software tool such as the Genome-to-Genome Distance Calculator 3.0 tool (https://ggdc.dsmz.de/ggdc.php; Meier-Kolthoff *et al.* 2022) to determine the distance between *L. paracasei* ALE6 and the genomes of other strains of the species, obtained from GenBank (Table 10).

**Table 10. distance between L. paracasei ALE6 and the genomes of other strains of the species**

| Microorganism | Genome | DDH¹ | DDH² | DDH³ | G+C dif |
|---|---|---|---|---|---|
| *L. paracasei* subsp. *tolerans* S-NB | NZ_CP068416.1 | 98.9 | 99.9 | 99.5 | 17 |
| *L. paracasei* subsp. *tolerans* S-NA5 | NZ_CP068408.1 | 98.9 | 99.9 | 99.5 | 17 |
| *L. paracasei* subsp. *tolerans* ZY-1 | NZ_CP065154.1 | 91.0 | 96.2 | 94.2 | 17 |
| *L. paracasei* ATG-E1 | NZ_CP110214.1 | 85.3 | 93.8 | 89.5 | 22 |
| *L. paracasei* CLP-C10 | NZ_CP052936.1 | 80.7 | 90.4 | 85.3 | 25 |
| *L. paracasei* subsp. *paracasei* 8700:2 | NC_022112.1 | 80.4 | 87.6 | 84.5 | 15 |
| *L. paracasei* subsp. *tolerans* MGB0625 | NZ_CP064311.1 | 79.7 | 87.7 | 83.9 | 24 |
| *L. paracasei* GM-080 | NZ_CP095406.1 | 79.4 | 87.4 | 83.6 | 25 |
| *L. paracasei* subsp. *tolerans* FX-6 | NZ_CP109944.1 | 78.7 | 87.4 | 83.1 | 17 |
| *L. paracasei* subsp. *paracasei* JCM 8130 | NZ AP012541.1 | 78.0 | 87.5 | 82.5 | 5 |

| | | | | | |
|---|---|---|---|---|---|
| ¹Formula 1 (%): length of all high-scoring segment pairs (HSPs) divided by total genome length. ²Formula 2 (%): sum of all identities found in HSPs divided by overall HSP length. ³Formula 3 (%): sum of all identities found in HSPs divided by total genome length. | | | | | |

Both studies show a small distance between *L. paracasei* ALE6 and other microorganisms of the *L. paracasei* species, with the greatest similarity (ANI and DDH) being with representatives of the subspecies *L. paracasei* subsp *tolerans.*

### Virulence factors

The presence of potential virulence factors in the *L. paracasei* ALE6 genome was assessed in silico using a software database tool, such as the online resource VFDB (http://www.mgc.ac.cn/cgi-bin/VFs/v5/main.cgi; Liu et al. 2022). The result of this analysis did not identify any sequences encoding toxins or that could be unambiguously related to pathogenic characteristics.

### Antibiotic resistance genes

The *L. paracasei* ALE6 genome was subjected to *in silico* analysis for sequences similar to antibiotic resistance genes and mutations related to resistance mechanisms. Two software database tools, such as the CARD (https://card.mcmaster.ca/about; Alcock *et al.* 2023) and ResFinder (http://genepi.food.dtu.dk/resfinder; Bortolaia *et al.* 2020; Camacho *et al.* 2009) tools and databases, were used to carry out this study, but none of the searches identified any genetic determinants related to antibiotic resistance in the *L. paracasei* ALE6 genome.

### EXAMPLE 2

### Identification of bacterial strains with antidiabetic effect in a diabetes model in C. elegans.

172 bacteria were tested in an age-1 mutant C. *elegans* model. Bacteria have been tested alive and thermally inactivated, at a concentration of 10% of the total feed. Greater proportions affected normal development of the nematode.

For the 172 samples, two main types of tests have been performed with the mutant age-1: reversal of entry into Dauer stage as diabetes model (B) and reversal of the phenotype of infertility as an effect of the reduction of signalling by the insulin route (A).

The regulation of entry into "dauer" is possibly a very restrictive condition and none of the samples tested has shown an effect related.

The first 108 bacteria were tested on the infertility model and the statistical analysis was performed by comparing the outcome of each bacterium with respect to the average result of all bacteria. Treating the data independently whether they are alive or thermally inactivated. In this case, a total of 13 bacteria that improved infertility when compared to average values were obtained. These bacterial samples are: BT97-alive, BT595-alive, BT679-IT, BT837-IT, BT834-IT, BT584-IT, BT577-IT and BT#819-IT and DES-405-IT, DES-0263IT, DES-0166-IT (ALE6), DES-379-live y DES-405-live. A case study of these can be observed in Figure 1.

Once this study has been carried out, an additional 64 bacteria were tested. For these only the infertility trial was performed, which is the one that had shown significant data. From these new 64 bacteria we observed that in 10 samples there was an effect of partial suppression of infertility. These samples are: DES-0073V, DES-0073-IT, DES-0166V (ALE6), DES-0190-IT, DES-0288-alive, DES-303-IT, DES-094-alive, DES-0356-IT, DES-0383alive y DES-0384-alive. (Figure 2)

In summary, the trials carried out indicate that from all the markers related to the insulin route in *C.elegans,* the infertility associated with it, is the most sensitive. After testing 172 bacterial samples, 14 samples have been shown to improve, in a statistically significant way, the infertility associated with the reduction of the insulin route signaling pathway, therefore they could be interesting for administration to humans, for example as a probiotic.

Strain DES-0166 corresponding to *Lacticaseibacillus paracasei* subsp. *tolerans* ALE6 (CECT 9458) was used for further analysis.

### EXAMPLE 3

### Evaluation of the effect of administration of Lacticaseibacillus paracasei subsp. tolerans ALE6 (CECT 9458) in an experimental model of metabolic syndrome in mice.

The experiments were performed with the bacterial (optionally probiotic) strain identified as *Lacticaseibacillus paracasei* subsp. *tolerans* ALE6 (CECT 9458) and consisted of feeding the experimental animals with a diet in which 60% of the caloric intake is provided by fat of animal origin. This is a model widely used for the preclinical validation of effective treatments of metabolic syndrome. In general, it is characterized by animal weight gain, significant increase in body adipose tissue, and is accompanied by metabolic alterations, characteristics of the situation that occurs in humans with metabolic syndrome.

### Methods

The study was carried out in accordance with the regulations included in the guide for the "Care and Use of Laboratory Animals" prepared by the Federation of European Laboratory Animal Science Associations (FELASA), and the protocols were approved by the Experimental Animal Ethics Committee of the University of Granada (Ref. No. 28/03/2016/030). The product to be tested; *Lacticaseibacillus paracasei* subsp. *tolerans* ALE6, was supplied by Biosearch S.A.U., a Kerry Company.

Male C57BL/6J mice (5-7 weeks old) obtained from Charles River Laboratories (Shrewsbury, MA, USA) were housed in makrolon cages, maintained in an air-conditioned atmosphere (22 ± 1°C, 55 ± 10% relative humidity) with a 12-hour light/dark cycle, and provided with a free access to tap water. Mice were fed with either a standard chow diet (13% calories from fat, 20% calories from protein and 67% calories from carbohydrate) (Global diet 2014; Harlan Laboratories, Barcelona, Spain) or a high-fat diet (HFD) (59% calories from fat, 13% calories from protein and 28% calories from carbohydrate) (Purified diet 230 HF; Scientific Animal Food & Engineering, Augy, France). Mice were randomly divided into different groups (n=10): non-obese (control diet), obese (HFD) and obese-treated (HFD-L.p). HFD-L.p mice were daily administered the bacterial strain *L. paracasei* subsp *tolerans* ALE6 (CECT 9458) at 5×10⁸ CFUs/mouse and control and obese mice received PBS.

After 11 weeks on their respective diets and treatments, animals were fasted for 8 hours and euthanized via isoflurane anaesthesia. Blood samples were collected by cardiac puncture in ice-cold tubes containing heparin and centrifuged at 5000 g at 4°C for 20 minutes, and the plasma was frozen at -80°C. Liver, colon, adipose tissue (abdominal and epididymal) and aorta were dissected, and weighed. Weight indices were calculated by dividing their weights by the tibia length. All tissue samples were frozen in liquid nitrogen and then stored at -80°C until analysis. All animal care was carried out in accordance with the 'Guide for the Care and Use of Laboratory Animals' as promulgated by the National Institute of Health, and the protocols approved by the Ethic Committee of Laboratory Animals of the University of Granada (Spain) (Ref. No. 28/03/2016/030).

### Glucose Tolerance Test

One week before the sacrifice, a glucose tolerance test was performed on mice fasted for 8 h. Fasting plasma glucose was measured by blood drops from the tail tip using a handheld glucometer (Contour XT, Ascensia Diabetes Care, S.L., Barcelona, Spain), followed by intraperitoneal injection of 50% glucose solution in water at 2 g/kg of body weight. Blood glucose values of the tail tip were measured at 15, 30, 60, and 120 minutes after glucose injection, and the area under the glucose curve of each mouse was calculated.

### Biochemical Analyses

Plasma glucose, LDL (low-density lipoprotein)-cholesterol and HDL (high-density lipoprotein)-cholesterol concentrations were measured by colorimetric methods using Spinreact kits (Spinreact, S.A., Girona, Spain). Plasma insulin concentrations were quantified using a mouse insulin ELISA kit (Alpco Diagnosis, Salem, NH, USA). Homeostatic model assessment of insulin resistance (HOMA-IR) was calculated using the formula: fasting glucose (mM)×fasting insulin (µU/mL)/22.5. Plasmatic LPS levels were quantified by the PierceTM Chromogenic Endotoxin Quant Kit (Thermo Scientific, Inc., Waltham, MA, USA) following manufacturer's instructions. Previously, plasma samples were treated for removing residual heparin used during blood extraction and fat with a 10 mM MgCl2 solution and PyrosperseTM Dispersing Agent (Lonza, Walkersville, MD, USA), respectively. Sterile and pyrogen-free material was always used to guarantee sample and test integrity.

Lipid peroxidation in the liver was evaluated by measuring thiobarbituric acid reactive substances (TBARS) that are formed as fat-degradation by-products. After liver lysis in RIPA lysis buffer containing proteases inhibitors, aldehydes from protein carbonyl groups react with thiobarbituric acid (TBA) forming an aldehyde-TBA complex, which can be detected by spectrophotometry (530 nm). Protein concentration was measured by the BCA method using BSA as standard.

### Histological Studies

Samples of epididymal adipose tissue and liver were fixed in 4% PFA, embedded in paraffin, and 5 µm-thick sections were taken. Then these sections were stained with hematoxylin and eosin. In addition, liver sections were fixed in 30% sucrose, embedded in OCT compound (Tissue-Tek^{®} O.C.T. Compound, Sakura^{®} Finetek, United States), and frozen with isopentane at -40 °C. Then, 8 µm-thick sections were taken and stained with oil red and hematoxylin. Area from adipocytes (in squared micrometers) was measured and analyzed using Fiji imaging software with the Adiposoft v1.16 plugin.

### Analysis of gene expression by RT-qPCR

Total RNA from liver, adipose tissue, colon and aortic rings samples as well as miRNAs from adipose tissue were extracted and reverse transcribed. mRNA and miRNA expression were analyzed by real-time PCR amplification using specific primers and was normalized using the Glyceraldehyde 3-phosphate dehydrogenase (GAPDH) and small nucleolar RNA C/D box 95 (SNORD95), respectively. Fold increases in values for gene expression analysis were calculated by the comparative Ct (ΔΔCt) method.

### Vascular reactivity studies and NADPH Oxidase Activity

Descending thoracic aortic rings were dissected from animals and were used to determinate the vasorelaxant ability of acetylcholine and NADPH oxidase activity as obesity-associated vascular dysfunction parameters.

### Microbiota analysis: DNA extraction and Illumina MiSeq sequencing

Faecal contents were collected from all animals at end point and total DNA was isolated and analyzed in order to characterize the microbiota composition as previously described (Rodriguez-Nogales et al 2017).

All results are expressed as the mean ± SEM. Differences between means were tested for statistical significance using a one-way analysis of variance (ANOVA) and post-hoc least significance tests. Differences between proportions were analysed with the chi-squared test. All statistical analyses were carried out with the GraphPad 5.0 software package (GraphPad Software, Inc., La Jolla, CA, USA), with statistical significance set at P< 0.05.

For microbiota evaluation, RDP was used for analyses of all sequences. The output file was further analysed using SPSS Statistics 17.0 Software Package (SPSS Inc., Chicago, IL,USA), Statistical Analysis of Metagenomic Profiles (STAMP) software package version 2.1.3 and Quantitative Insights Into Microbial Ecology (QIIME) software (Knight Lab, San Diego, CA, USA) (Version 1.9.1).

### Results

Administration of *L. paracasei* subsp *tolerans* ALE6 (CECT 9458) reduced body weight gain and improved glucose tolerance test and plasma biochemical profile.

Administration of *L. paracasei* subsp *tolerans* ALE6 (CECT 9458) significantly reduced body weight gain in treated obese mice from day 43 onwards, without modifying total energy intake, but lowering energy efficiency compared with untreated obese mice (Figure 3).

The impact of administration of *L. paracasei* subsp *tolerans* (CECT 9458) on body weight was associated with significant reductions of both epididymal and abdominal fat deposits (Figure 4). Additionally, when the glucose tolerance test was performed, mice fed HFD showed significantly higher glucose level peaks than those fed control diet, as a marker of glucose metabolism impairment in obese mice; however, administration of *L. paracasei* subsp *tolerans* (CECT 9458) resulted in an improvement in glucose tolerance since it significantly reduced plasma glucose concentrations from 60 minutes onwards, compared with non-treated mice, and evidenced by a significant reduction in the area under the curve (AUC) (Figure 5A). Accordingly, at the end of the study, fasting glycaemia and insulin levels in HFD-fed mice were significantly higher in control obese mice than in *L*. *paracasei*-treated mice, which was similar to control diet-fed mice. As a result, *L. paracasei* CECT improved insulin sensitivity as evidenced by a lower HOMA-IR index value compared to HFD-fed mice (Figure 5B).

Regarding to the plasma lipid profile was also altered in control HFD-fed mice, with an increased LDL/HDL ratio, which was significantly reduced after the supplementation of *L*. *paracasei* to obese mice (Figure 6).

The histological analysis of epididymal fat tissue from untreated HFD-fed mice showed hypertrophy when compared with control diet-fed mice, which was significantly ameliorated after administration with *L. paracasei* (Figure 7). Hyperplasia process involves several hormones and transcription factors, including sterol regulatory element-binding factor 1 (Srebf1), CCAAT/enhancer-binding protein alpha (Cebpa), peroxisome proliferator-activated receptor gamma (Pparg) and fatty acid-binding protein 4 (Fabp4). In fact, our results show that mRNA expression of these markers was significantly increased in the adipose tissue from control obese mice when compared with the non-obese group, which were ameliorated after the administration of *L. paracasei* to obese mice (Figure 8).

The association between the increase in adipose tissue mass and the presence of a subclinical inflammatory state in obesity is well known. In the present study, this inflammatory state was characterized by an increased expression of pro-inflammatory cytokines genes, including TNF-alpha and IL-6, as well as the chemokine monocyte chemotactic protein-1 (Mcp1) in fat tissue, thus leading to the activation of different inflammatory pathways, such as the c-jun N-terminal kinase (JNK)-related signaling pathway, which has been reported to interfere with insulin signaling and the subsequent impaired glucose metabolism (Figures 9). The administration of *L*. *paracasei* to obese mice significantly reduced the increased expression of these inflammatory markers to similar levels of that obtained in control diet-fed mice. Similarly, the upregulated expression of Jnk1 observed in adipose tissue of control HFD-fed mice was reduced with the administration of *L. paracasei* subsp *tolerans* (CECT 9458) (Figure 9). In consequence, the ability of *L. paracasei* (for example, *L. paracasei* subsp *tolerans, such as the specific bacterial strain* deposited *as* CECT 9458) to ameliorate the obesity-associated inflammatory status could contribute to the improvement of insulin signaling and glucose metabolism in obese mice.

The peroxisome proliferator-activated receptor alpha (PPARα) is considered a transcription factor which appears to play a crucial role in intracellular lipid metabolism. HFD group mice displayed a reduction on the expression of PPARalpha gene on adipose tissue compared with control diet group, and was significantly ameliorated after administration of *L. paracasei* subsp *tolerans* (CECT 9458) (Figure 10), insinuating an improvement in lipid profile.

Besides, white adipose tissue is recognized as a dynamic endocrine organ able to produce and release several bioactive polypeptides known as adipokines which includes adiponectin and leptin. On the one hand, adiponectin is considered an anti-inflammatory molecule which acts as an insulin sensitizer through AMP protein kinase (AMPK), a cell nutrient sensor which has been proposed as a link between nutrient metabolism and inflammation in target tissues. On the other hand, leptin in addition to regulating energy expenditure and appetite, also opposes insulin action. In obesity, there is an imbalance in the profile of these adipokines which is characterized by an increase in leptin levels and a reduction in adiponectin, in combination with a decreased expression of Ampk and leptin receptor both in liver and adipose tissue. These observations were confirmed in HFD-fed mice, where the expression of Adipoq, Lepr and Ampk genes was decreased in liver and/or adipose tissue and associated with an elevated fat expression of Lep gene. Of note, the administration with *L. paracasei* significantly ameliorated the expression of all these markers (Figure 10).

Recently, numerous studies have pointed that miRNAs play an important role in adipocyte differentiation and contribute to the development of obesity, metabolic syndrome, PCOS. Therefore, their imbalance may play a role in the development of obesity and related metabolic complications. A recent study has highlighted that the expression of miR-221 is up-regulated in obese people and that it can modulate fat metabolism through leptin and TNF-α. Interestingly, an increase in the expression of miR-221 in the adipose tissue and liver from different obesity models has also been reported. It has been also demonstrated the up-regulation of miR-142-3p, miR-142-5p, miR-146a and miR-146b during the development of obesity in mice and seems to be closely related to the increase in the pro-inflammatory cytokine TNF-α. Similarly, a pro-inflammatory connection between miR-155 and NF-κB has been described, which could participate in the amplification of the inflammatory status in adipocytes typically found in obesity. Furthermore, miR-375 has been reported to promote the differentiation of 3T3-L1 adipocytes by up-regulating the expression of C/ebp and Ppary genes. It has also been suggested that miR-9 are capable of regulating insulin pathways through sirtuin 1 (SIRT1), a nicotinamide adenosine dinucleotide (NAD) that regulates energy homeostasis, inflammation, and metabolic disease associated with obesity. miR-146a and miR-155 are also upregulated in women suffering PCOS. In the animal model, non-treated obese mice displayed significant increased levels of miR-221, miR-142-3p, miR-142-5p, miR-146a, miR-146b, miR-155, miR-375 and miR-9 in adipose tissue when compared to control diet-fed mice and were restored with *L. paracasei* administration a exception of miR-375 and miR-9 (Figure 11).

It is widely accepted that gut microbiota composition is altered in obese individuals, manifesting reduced gut bacterial diversity, richness and evenness. As previously reported, non-treated obese mice showed a significant reduction of alpha diversity measured by Shannon index (a richness and evenness estimator), Chao1 index (diversity estimation), Observed OTUs (count of unique OTUs in each sample) and Phylogenetic diversity (PD) whole tree (consider the phylogeny to estimate diversity across a tree) when compared with lean mice. However, administration of *L. paracasei* to obese mice achieved a significant increase in all the above-mentioned parameters (Figure 12).

The relative abundance of gut microbiota at phylum and genus level between treated and untreated mice was also investigated. It is well known that obese people manifest lower proportions of Bacteroidetes (B) and higher abundance of Firmicutes (F) than those without obesity. This imbalance leads to an increased F/B ratio, which is reported to increase energy harvesting from the diet, regulate fat storage as well as modulate the formation of substrates for storable fat synthesis. Besides, alterations in microbiota composition produce compounds absorbed into the systemic circulation that are capable of contributing to the onset of obesity-associated comorbidities, since they increase both insulin resistance and inflammatory tissue damage. These findings were confirmed in our study where non-treated obese mice showed an imbalance in intestinal microbiota, with higher F/B ratio than control diet-fed mice. However, *L*. *paracasei* administration was able to counteract the altered composition in the gut microbiota restoring the main bacteria phyla to the typical values observed in control diet-fed mice (Figure 13). Similarly in women suffering PCOS a reduction of alpha diversity and increased F/B ratio due to lower proportion of Bacteroidetes.

In the last years, *Akkermansia muciniphila* have become the focus of growing attention since its abundance is inversely related to body weight and type 2 diabetes. Previous studies have reported that *A*. *muciniphila* treatment, or those that promote its abundance, could reverse HFD-induced metabolic disorders and other insulin resistance related conditions as PCOS. Inversely, *Clostridium* spp, also known to degrade some animal mucin, has been positively associated with metabolic syndrome and. Our data confirmed these findings since HFD mice showed increased proportions of *Clostridium* while reduced *Akkermansia* when compared with lean mice. Furthermore, the relative Lactobacillus abundance was also reduced in obese mice, thus limiting the potent effects of this bacteria on several key transcriptional and translational factors in adipose tissue. Once again, administration of *L. paracasei* to HFD-fed mice reached to restore obesity-associated gut dysbiosis (Figure 13).

### EXAMPLE 4

### Evaluation of the synergistic effect of administration of Lacticaseibacillus paracasei subsp. tolerans ALE6 (CECT 9458) and D-chiroinositol in an experimental model of metabolic syndrome in mice.

The experiments were performed with D-chiroinositol and the probiotic strain identified as *Lacticaseibacillus paracasei* subsp. *tolerans* ALE6 (CECT 9458) and consisted of feeding the experimental animals with a diet in which 60% of the caloric intake is provided by fat of animal origin. This is a model widely used for the preclinical validation of effective treatments of metabolic syndrome. In general, it is characterized by animal weight gain, significant increase in body adipose tissue, and is accompanied by metabolic alterations, characteristics of the situation that occurs in humans with metabolic syndrome.

### Methods

The study was carried out in accordance with the regulations included in the guide for the "Care and Use of Laboratory Animals" prepared by FELASA, and the protocols were approved by the Experimental Animal Ethics Committee of the University of Granada (Ref. No. 94-CEEA-OH 2015). The product to be tested; *Lacticaseibacillus paracasei* subsp. *tolerans* ALE6 and D-chiroinositol was supplied by Biosearch S.A.U., a Kerry company.

Male C57BL/6 mice, 7-9 weeks old and weighing 20-25 g, were used and supplied by Charles River (France). The mice were randomly divided into 3 experimental groups: lean control group (standard diet), obese control group (high fat diet) and an obese group that were treated daily orally with *Lacticaseibacillus paracasei* subsp. *tolerans* (5·10⁸ cfu). During the experimental period (5 weeks) body weight, food and drink consumption were measured weekly. At the end of the treatment, and before sacrificing the mice, plasma samples were taken. Next, different tissues were obtained for macroscopic assessment (liver, heart, kidneys, brain, spleen, lungs, small intestine, cecum and large intestine) as well as abdominal and epididymal fat, in which different markers related to the metabolic syndrome were assessed. For metabolic characterization, plasma levels of glucose, t and cholesterol (cHDL and cLDL) were measured by spectrophotometric techniques, as well as insulin, which was calculated by enzyme-immunoassay. The degree of insulin resistance was evaluated with the HOMA-IR calculation. Adipose tissue samples were used to extract RNA in order to evaluate the expression of different biomarkers that are altered in obesity conditions: Inflammatory markers (TNF-α, IL-β, IL-6), enzymes related to intracellular signaling pathways (JNK-1 and JNK-2), proteins involved in the maintenance of energy homeostasis (leptin and adiponectin), peroxisome proliferator-activated receptor (PPAR), glucose membrane transport protein (GLUT-4) and AMP protein kinase (AMPK), all of which involved in metabolic processes.

Results are expressed as mean ± SEM. Statistical differences between the means were established by ANOVA, and by the Bonferroni test when necessary. Statistical analysis of the results was carried out using GraphPad Software 6.0 (GraphPad Software Inc., La Jolla, CA, USA), establishing statistical significance at p < 0.05 (in the different graphs included in this report, the groups with different letters show significant differences).

### Results

Data related to the evolution of the weight of the animals during the 5 weeks revealed that the mice fed a diet high in fat (diet induced obesity -DIO-) showed a significantly higher weight compared to the group that received the normal diet (Lean control). Obese mice under treatment DCI or DCI+PROB showed a significant reduction in this weight gain, although no significant differences between both treatments are observed (Figure 14). As no differences in energy consumption were observed between experimental groups that received the high-fat diet, the effect would be related to the decrease in energy efficiency observed (Figure 15).

The evaluation of the state of insulin resistance revealed that the baseline blood glucose values were significantly higher in the obese control group than the lean group, obtaining a significant reduction in the obese groups undergoing both treatments, DCI and DCI+PROB (Figure 16A). The calculation of the HOMA-IR parameter showed that the obese group presented significantly increased values with respect to the lean group, indicating a situation of insulin resistance characteristic of the obesity. Although level of HOMA-IR was lower in both treatments values did not reach statistical significance versus the obese group (Figure 16B). It is hypothesized that time of intervention was too short to observe differences as probiotic treatment significantly reduced HOMA-IR after 11 weeks of intervention in a similar model (Example 3).

The evaluation of the plasma lipid profile showed that, compared to the lean group, the obese animals showed a significant increase in the levels of LDL/HDL cholesterol ratio. Obese group that received both treatments, DCI and DCI+PROB, showed a tendency to reduce these increased values (Figure 17).

Macroscopic analysis of adipose (abdominal and epididymal) tissue revealed that obese mice in the control group showed a significant increase in weight compared to the lean control group. Both treatments, DCI and DCI+PROB trended to promote a reduction in the weight of the content of the two types of fat (Figure 18).

In addition to metabolic disorders, obesity is associated with systemic inflammation, which affects adipose tissue; so that mice fed DIO showed increased mRNA expression of the pro-inflammatory cytokines IL-1β, TNFα and IL-6 compared to the lean group. DCI trended to reduce these pro-inflammatory cytokines but a better anti-inflammatory effect was observed in DCI+PROB group as it induced a significant reduction in IL-1β and IL-6 up to lean control levels (Figure 19).

On the other hand, leptin, adipokine secreted by adipose tissue, shows a key role in the integration of systemic metabolism, its production and function being altered in obesity. In the present study, the expression of leptin in adipose tissue was affected in obese mice when compared to mice fed a standard diet. DCI was not able by itself to significantly reduce leptin, however, the combination of DCI+PROB normalize the level of this adipokine (Figure 20).

In the present study, the expression of PPARγ in adipose tissue was significantly reduced in obese mice when compared to mice fed a standard diet. PPARγ is ligand-mediated transcription factor mainly expressed in adipose tissue, macrophages, vascular smooth muscle. PPARγ had a key role in regulating adipocyte differentiation and insulin sensitivity. Therefore, modulators of PPARγ activity have the potential for the treatment of type 2 diabetes. In fact, PPARγ full agonists-glitazone class drugs (TZD) such as pioglitazone and rosiglitazone-are used for the treatment of type 2 diabetes and also for PCOS treatment. Despite the beneficial clinical effects of these drugs, TZDs cause several adverse effects, including significant weight gain, peripheral edema, bone loss, and an increased risk of congestive heart failure, mainly arising from their over-activation of PPARγ. Thus, novel PPARy-modulating agents that normalize PPARγ moderately would provide a safe alternative avoiding the side effects related to over-activation. DCI treatment did not affect PPARγ level, however, the combination of DCI+PROB restored its level (Figure 21).

### EXAMPLE 5

**Evaluation of the effect of a combination of D-chiro-inositol and** *Lacticaseibacillus* ***paracasei* subsp. *tolerans* ALE6 (CECT 9458) on glycemic metabolism in women with polycystic ovary syndrome (PCOS) and insulin resistance.**

### Methods

This was a randomized, parallel, double-blind, placebo-controlled study. Study participants met the following inclusion criteria: PCOS women > 18 years old according to the Rotterdam criteria with a body mass index (BMI) <35 kg/m2 and a HOMA index ≥ 1.5 that do not require pharmacological treatment. Exclusion criteria were women on antidiabetic drugs, being pregnant or intending to get pregnant, being in fertility treatment, taking probiotics or antibiotics, or suffering from any disease that affects the development and results of the study.

A total of 41 subjects was enrolled and randomly assigned into two different groups: one group received daily 500 mg of D-chiro-inositol and 1 x 10⁹ cfu of the probiotic *Lacticaseibacillus paracasei* subsp. *tolerans* ALE6 (DCI-prob group) and the other received maltodextrin (Control group). Treatments were given to subjects for 12-weeks.

The primary outcome of the study was the changes in the insulin resistance through the determination of the homeostatic model assessment (HOMA-IR). HOMA-IR was calculated as (fasting insulin in mIU/L x fasting glucose in mg/dL)/405. Secondary outcome was weight and the body mass index (BMI).

### Results

The HOMA-IR significantly decreased in the group that consumed the DCI-Prob (p-time =0.040), whereas did not change in the placebo group (p-time=0.417) (Figure 22).

Regarding to BMI, at the end of the intervention, the BMI trended to be lower in the DCI-Prob than in the control group (p=0.074) (Figure 23A). When population was distributed in normal weigh (BMI 18,5-24.9), overweight (BMI 25-29.9) and obese (BMI ≥30) a significant lower BMI was observed in DCI-Prob group versus control group (Figure 23B).

### References

Alcock BP, Huynh W, Chalil R, Smith KW, Raphenya AR, Wlodarski MA, Edalatmand A, Petkau A, Syed SA, Tsang KK, Baker SJC, Dave M, McCarthy MC, Mukiri KM, Nasir JA, Golbon B, Imtiaz H, Jiang X, Kaur K, Kwong M, Liang ZC, Niu KC, Shan P, Yang JYJ, Gray KL, Hoad GR, Jia B, Bhando T, Carfrae LA, Farha MA, French S, Gordzevich R, Rachwalski K, Tu MM, Bordeleau E, Dooley D, Griffiths E, Zubyk HL, Brown ED, Maguire F, Beiko RG, Hsiao WWL, Brinkman FSL, Van Domselaar G, McArthur AG. 2023. "CARD 2023: expanded curation, support for machine learning, and resistome prediction at the Comprehensive Antibiotic Resistance Database". Nucleic Acids Res. Jan 6;51(D1):D690-D699.
Altschul, S.F., Gish, W., Miller, W., Myers, E.W., Lipman, D.J. 1990. "Basic local alignment search tool." J. Mol. Biol. 215:403-410.
Berthier, F., and S. D. Ehrlich. 1998. "Rapid species identification within two groups of closely related lactobacilli using PCR primers that target the 16S/23S rRNA spacer region". FEMS Microbiol. Lett. 161:97-106.
Bortolaia V, Kaas RS, Ruppe E, Roberts MC, Schwarz S, Cattoir V, Philippon A, Allesoe RL, Rebelo AR, Florensa AR, Fagelhauer L, Chakraborty T, Neumann B, Werner G, Bender JK, Stingl K, Nguyen M, Coppens J, Xavier BB, Malhotra-Kumar S, Westh H, Pinholt M, Anjum MF, Duggett NA, Kempf I, Nykäsenoja S, Olkkola S, Wieczorek K, Amaro A, Clemente L, Mossong J, Losch S, Ragimbeau C, Lund O, Aarestrup F. M. 2020. "ResFinder 4.0 for predictions of phenotypes from genotypes". Journal of Antimicrobial Chemotherapy, 75(12),3491-3500
Camacho C, Coulouris G, Avagyan V, Ma N, Papadopoulos J, Bealer K, Madden TL. 2009. "BLAST+: architecture and applications". BMC Bioinformatics 10(1):421
International Standard Organization, 2012. ISO standard 10932:2012 "Milk and milk products: Determination of the minimal inhibitory concentration (MIC) of antibiotics applicable to bifidobacteria and non-enterococcal lactic acid bacteria (LAB)".
Liu B, Zheng DD, Zhou SY, Chen LH and Yang J, 2022. "VFDB 2022: a general classification scheme for bacterial virulence factors". Nucleic Acids Res. 50(D1):D912-D917
Meier-Kolthoff, J.P., Sardà Carbasse, J., Peinado-Olarte, R.L., Göker, M. 2022. "TYGS and LPSN: a database tandem for fast and reliable genome-based classification and nomenclature of prokaryotes". Nucleic Acid Res 50:D801-D807.
Reimer, L.C., Sardà-Carbasse, J., Koblitz, J., Ebeling, C., Podstawka, A., Overmann, J. 2022. "BacDive in 2022: the knowledge base for standardized bacterial and archaeal data". Nucleic Acids Research; database issue 2022.
Rychen, G., Aquilina, G., Wester, P., Bampidis, V., Bastos, M. de L., Bories, G., Chesson, A., Cocconcelli, P. S., Flachowsky, G., Gropp, J., Kolar, B., Kouba, M., López□Alonso, M., López Puente, S., Mantovani, A., Mayo, B., Ramos, F., Saarela, M., Villa, R. E., Galobart, J. 2018. "Guidance on the characterisation of microorganisms used as feed additives or as production organisms". EFSA Journal, 16(3).
Seemann T. 2014. "Prokka: rapid prokaryotic genome annotation". Bioinformatics 15;30(14):2068-9.
Ward, L. J. H., and M. J. Timmins. 1999. "Differentiation of Lactobacillus casei, Lactobacillus paracasei and Lactobacillus rhamnosus by polymerase chain reaction". Lett. Appl. Microbiol. 29:90-92
Yoon, S. H., Ha, S. M., Lim, J. M., Kwon, S.J. & Chun, J. 2017. "A large-scale evaluation of algorithms to calculate average nucleotide identity". Antonie van Leeuwenhoek. 110:1281-1286.

## Claims

1. A bacterial strain of *Lacticaseibacillus paracasei* subsp. *tolerans* deposited in the CECT under Accession N° 9458, or a variant thereof.

2. The bacterial strain according to claim 1; wherein the bacterial strain is alive.

3. The bacterial strain according to claim 1; wherein the bacterial strain is inactivated, optionally partially inactivated, optionally totally inactivated.

4. The bacterial strain according to claim 3; wherein the bacterial strain has been inactivated by an inactivation process selected from the group consisting of thermal inactivation, microwave inactivation, pressure inactivation, acid inactivation, base inactivation, ethanol inactivation and peroxide inactivation.

5. The bacterial strain according to any preceding claim; wherein the bacterial strain is a probiotic strain.

6. The bacterial strain according to any preceding claim; wherein the bacterial strain comprises a 16S rRNA gene sequence comprising a nucleotide sequence that is at least 90% identical to the nucleotide sequence of SEQ ID NO: 1.

7. A biologically pure culture, or a composition, or a pharmaceutical product, or a feed, or a nutritional product, or a nutraceutical composition; comprising the bacterial strain according to any one of claims 1 to 6.

8. The biologically pure culture, composition, pharmaceutical product, feed, nutritional product, or nutraceutical composition according to claim 7; further comprising D-chiro-inositol.

9. The bacterial strain according to any of claims 1 to 6, or the biologically pure culture, composition, pharmaceutical product, feed, nutritional product, or nutraceutical composition according to claim 7 or 8; wherein the bacterial strain, or the biologically pure culture, composition, pharmaceutical product, feed, or nutritional product, is frozen, lyophilized or dried.

10. A bacterial strain of *Lacticaseibacillus paracasei* subsp. *tolerans* for use as a medicament.

11. The bacterial strain for use according to claim 10; wherein the bacterial strain is the bacterial strain according to any of claims 1 to 6.

12. The bacterial strain for use according to claim 10 or 11, wherein the bacterial strain is combined with D-chiro-inositol.

13. The bacterial strain for use according to any of claims 10 to 12; wherein the bacterial strain is for use in the treatment and/or prevention of insulin resistance related infertility in a female subject, optionally wherein the insulin resistance related infertility is associated with diabetes, polycystic ovary syndrome and/or with insulin resistance syndrome; or wherein the bacterial strain is for use in the treatment and/or prevention of a condition selected from the group consisting of: gestational diabetes, insulin resistance syndrome, type 2 diabetes, obesity, heart disease, high blood pressure, proinflammatory state, proinflammatory condition, and polycystic ovary syndrome.

14. The bacterial strain for use according to any of claims 10 to 13; wherein the bacterial strain is comprised in the biologically pure culture, composition, pharmaceutical product, feed, nutritional product, or nutraceutical composition according to any of claims 7 to 9.

15. A cosmetic method for the treatment of obesity comprising the administration to a subject of the bacterial strain according to any of claims 1 to 6 or 9, or the biologically pure culture, composition, feed, nutritional product, or nutraceutical composition, according to any one of claims 7 to 9.
